# EUROPEAN PATENT APPLICATION

(11) **EP 4 624 498 A1**
(43) Date of publication of application: **01.10.2025**
(21) Application number: 23893995.3
(22) Date of filing: 24.11.2023
(51) Int. Cl.: C07K 19/00, C12N 15/11, A61K 38/20, A61K 35/00

(54) **FUSION POLYPEPTIDE AND USE THEREOF**

(30) Priority: 25.11.2022 WO PCT/CN2022/134273; 31.03.2023 WO PCT/CN2023/085351
(71) Applicant: Suzhou Grit Biotechnology Co., Ltd., Shanghai 200120 (CN); Zhuhai Tuoyu Biotechnology Co., Ltd., Shanghai 200120 (CN); Shanghai Grit Biotechnology Co., Ltd., Shanghai 200120 (CN); Shenzhen Grit Biotechnology Co., Ltd., Shanghai 200120 (CN)
(72) Inventor: LIU, Yarong, Shanghai 200120 (CN); SUN, Jingwei, Shanghai 200120 (CN); WANG, Jingman, Shanghai 200120 (CN); JIN, Jiahui, Shanghai 200120 (CN)
(74) Representative: Cabinet Becker et Associés
(86) International application number: PCT/CN2023/133821
(87) International publication number: WO 2024/109906

(57) **Abstract**

The present invention provides a fusion polypeptide, specifically a fusion polypeptide comprising IL-15, as well as a method of preparing cells expressing the fusion polypeptide of IL-15 and uses thereof.

## Description

### Technical Field

The present invention relates to the field of biomedicine, and specifically to a fusion polypeptide and its uses thereof.

### Background

IL-15 is a cytokine that plays a key role in immune responses. For example, IL-15 can promote the proliferation of immune cells. Normally, IL-15 can be combined with its receptor protein IL-15Rα to more effectively activate the IL-15 receptor signal. Then IL-15 is connected to IL-15Rα in the form of a fusion protein or expressed on the immune cell membrane. However, the existing IL-15-IL-15Rα fusion proteins still have certain limitations in the applications in the field of immune cell therapy, such as short-lasting and unstable expression on cells, and the limited proliferation ability and function improvements of the cell population after expressing the fusion protein.

Therefore, there is an urgent need in this field for a new IL-15-IL-15Rα fusion protein to enable IL-15 to exert a longer-lasting effect and/or to enable the IL-15-IL-15Rα-expressing cell population to produce stronger functions.

### Summary of the Invention

In a first aspect, the present invention provides a fusion polypeptide, and immune cells expressing the fusion polypeptide of the present invention can have characteristics selected from the following group: (1) increased cell proliferation ability; (2) enhanced cytokine secretion ability; (3) improved cell killing ability; (4) improved continuous cell killing ability; (5) improved cell viability; (6) improved administration safety; (7) improved ability to inhibit tumor growth in vivo; (8) optimized cell phenotypic properties. For example, the invention provides a fusion polypeptide comprising a mutated interleukin-15 (IL-15) receptor or a functional fragment thereof compared to a protein comprising a native interleukin-15 receptor. After the IL-15 receptor is expressed in a cell, the fraction of membrane-bound expression product in the cell increases.

In another aspect, the invention provides a nucleic acid encoding the fusion polypeptide of the invention.

In another aspect, the invention provides a vector comprising the nucleic acid of the invention.

In another aspect, the invention provides a cell comprising the fusion polypeptide of the invention, the nucleic acid of the invention and/or the vector of the invention.

In another aspect, the present invention provides a method for preparing the fusion polypeptide of the present invention, which method includes culturing the cells of the present invention under conditions that allow the expression of the fusion polypeptide.

In a second aspect, the present invention provides a linker peptide, a fusion protein comprising the linker peptide, and/or immune cells expressing the fusion protein. The immune cells of the present invention have one or more of the following characteristics: (1) increased cell proliferation ability; (2) enhanced cytokine secretion ability; (3) increased cell killing ability; (4) improved or stable transduction efficiency of exogenous genes; and/or (5) increased functions for downstream signaling pathway, for example, STATS phosphorylation level. For example, the present invention provides a linker peptide that contains a number of strongly hydrophilic amino acids in a proportion of about 15% to 40% of the total number of all amino acids in the linker peptide. For example, the strongly hydrophilic amino acids include amino acids have normalized hydrophobicity number less than -0.4. For example, strongly hydrophilic amino acids include Glu(E), Asn(N), Gln(Q), Asp(D), Lys(K), and Arg(R).

In another aspect, the present invention provides a fusion protein comprising the linker peptide of the present invention.

In another aspect, the present invention provides a method for preparing the fusion protein of the present invention, which includes linking two or more fragments of the fusion protein through the linker peptide of the present invention.

In another aspect, the present invention provides the use of the above-mentioned linker peptide in producing fusion proteins.

In another aspect, the present invention provides cells comprising the above-mentioned linker peptide and/or fusion protein.

In another aspect, the invention provides a composition comprising the fusion polypeptide/fusion protein of the invention, the nucleic acid of the invention, the vector of the invention, and/or the cell of the invention, and optionally a pharmaceutical acceptable carrier.

In another aspect, the invention provides a kit comprising the fusion polypeptide/fusion protein of the invention, the nucleic acid of the invention, the vector of the invention, the cell of the invention, and/or the composition of the invention.

In one aspect, the invention provides a method for stimulating an immune response, comprising administering the fusion polypeptide/fusion protein of the invention, the nucleic acid of the invention, the vector of the invention, the cell of the invention, the composition of the invention and/or the kit of the invention.

In one aspect, the invention provides the use of the fusion polypeptide/fusion protein of the invention, the nucleic acid of the invention, the vector of the invention, the cell of the invention, the composition of the invention and/or the kit of the invention in the preparation of a medicament to prevent, alleviate and/or treat diseases. Preferably, the disease includes tumors.

In one aspect, the invention provides a method for preventing, alleviating and/or treating diseases, comprising administering the fusion polypeptide/fusion protein of the invention, the nucleic acid of the invention, the vector of the invention, the cell of the invention, and the combination of the invention and/or kits of the present invention. Preferably, the disease includes tumors.

In one aspect, the invention provides the fusion polypeptide/fusion protein of the invention, the nucleic acid of the invention, the vector of the invention, the cell of the invention, the composition of the invention and/or the kit of the invention for use in preventing, alleviating and/or treating diseases. Preferably, the disease includes tumors.

Other aspects and advantages of the present invention will be readily apparent to those skilled in the art from the detailed description that follows. Only exemplary embodiments of the invention are shown and described in the following detailed description. As those skilled in the art will realize, the contents of the present invention enable those skilled in the art to make changes to the disclosed specific embodiments without departing from the scope of the invention to which the present invention relates. Accordingly, the drawings and description of the present invention are illustrative only and not restrictive.

### Description of the Drawings

The features and advantages of the invention to which the present invention relates can be better understood by reference to the exemplary embodiments described in detail below and the accompanying drawings. A brief description of the drawings is as follows:
FIG. 1 shows the mbIL-15 transduction efficiency of T cells in each group.
FIG. 2 shows the proliferation ability of T cells in each group.
FIGs. 3 and 4 show the secretion of cytokines by T cells in the medium group and CD3 antibody group.
FIG. 5 shows the proportion of viable cells of T cells in each group.
FIG. 6 shows the continuous killing of tumor cells by T cells in each group.
FIG. 7 shows the IL-15 secretion by T cells in each group.
FIGs. 8 and 9 show the tumor volume and body weight of mice in each group.
FIGs. 10 and 11 show the secretion of IL-15 in the peripheral blood of T mice in each group.
FIGs. 12 and 13 show the number and phenotype of T cells in the peripheral blood of mice in each group.
FIG. 14 shows that compared with the PC228 group transduced with the fusion protein of GS linker, the proliferation ability of T cells in each group transduced with the fusion protein of modified linker.
FIGs. 15A-15B show that compared with the PC228 and/or PC163 group transduced with the fusion protein of GS linker, the cytokine secretion ability of T cells transduced with the fusion protein of modified linker.
FIG. 16 shows the ability of each group of T cells transduced with mbIL-15 fusion protein to kill tumor cells.
FIG. 17 shows the transduction efficiency of mbIL-15 fusion proteins containing the linker peptide of the invention.
FIGs. 18A-18C show the proliferation ability of TILs transduced with mbIL-15 fusion protein.
FIGs. 19A-19B show the ability of TILs transduced with mbIL-15 fusion protein to secrete the cytokines IL-2 and IFN-γ.
FIG. 20 shows the cell killing ability of TIL cells transduced with mbIL-15 fusion protein.
FIGs. 21A-21B show that in the G-Rex24 culture system (FIG. 21A) or under the 500ml culture bottle (FIG. 21B), the mbEL-15 fusion protein containing the linker peptide of the present invention after transduction can maintain stable transduction efficiency.
FIG. 22 shows that compared with the PC163 group transduced with mbIL-15 fusion protein of GS linker, the ratio of CD8/CD4 T cells in TIL transduced with mbIL-15 fusion protein, the transduction efficiency, and the ratio between IL-15Rα negative and IL- STATS phosphorylation ratio of 15Ra-positive T cells.
FIG. 23A shows the total number of viable cells in each group of cells transduced with the fusion protein.
FIG. 23B shows the CD8 expression of each group of cells transduced with the fusion protein + T cell survival ability.
FIG. 23C shows the transduction efficiency of each group of cells transduced with the fusion protein.
FIG. 23D shows the proportion of Tcm of central memory T cells in each group of cells transduced with the fusion protein.
FIG. 23E shows the cell killing ability of each group of cells transduced with the fusion protein.
FIG. 23F shows the p-STATS signal levels of cells in each group transduced with the fusion protein.
FIG. 23G shows the cell proliferation ability of each group of cells transduced with the fusion protein.
FIG. 23H shows the cytokine release ability of each group of cells transduced with the fusion protein.
FIG. 24A shows the cell killing ability of each group of cells transduced with the fusion protein.
FIG. 24B shows the cytokine release ability of each group of cells transduced with the fusion protein.
FIG. 24C shows the surface marker expression of each group of cells transduced with the fusion protein.
FIG. 24D shows the cell survival ability of each group of cells transduced with the fusion protein.

### Specific Embodiments

The implementation of the present invention will be described below with specific examples. Those familiar with the technology can easily understand other advantages and effects of the present invention from the content disclosed in this specification.

### Definitions

In the present invention, the term "linker peptide" is generally used interchangeably with the term "linker". For example, a linker peptide is a peptide that covalently or non-covalently links two or more molecules or peptides, thereby creating a larger complex consisting of all molecules or peptides including the linker peptide.

In the present invention, the term "fusion protein" generally refers to a protein composed of two or more molecules or peptides, which are usually not combined in the natural state, but the respective head and ends can be directly or indirectly bound together to form a continuous polypeptide. For example, a portion of a fusion protein may be called the "first polypeptide". For example, another portion of the fusion protein may be referred to as the "second polypeptide." For example, a fusion protein may contain two or more parts, and the "first polypeptide" or "second polypeptide" does not limit the number of components of the fusion protein.

In the present invention, the term "hydrophilic amino acid" generally refers to natural or artificial amino acids with hydrophilic groups in their side chains. For example, a hydrophilic amino acid may refer to an amino acid that exhibits hydrophobicity <0 according to the normalized consensus hydrophobicity scale (Eisenberg et al., 1984, J. Mol. Biol. 179:125- 142). For example, a normalized common hydrophobicity scale for amino acids can be:

| Amino Acid Type | | | Normalized common hydrophobicity scale |
|---|---|---|---|
| R | Arg | Arginine | -2.53 |
| K | Lys | Lysine | -1.5 |
| D | Asp | Aspartic acid | -0.9 |
| Q | Gln | Glutamine | -0.85 |
| N | Asn | Asparagine | -0.78 |
| E | Glu | Glutamic acid | -0.74 |
| H | His | Histidine | -0.4 |
| S | Ser | Serine | -0.18 |
| T | Thr | Threonine | -0.05 |
| P | Pro | Proline | 0.12 |
| Y | Tyr | Tyrosine | 0.26 |
| C | Cys | Cysteine | 0.29 |
| G | Gly | Glycine | 0.48 |
| A | Ala | Alanine | 0.62 |
| M | Met | Methionine | 0.64 |
| W | Trp | Tryptophan | 0.81 |
| L | Leu | Leucine | 1.06 |
| V | Val | Valine | 1.08 |
| F | Phe | Phenylalanine | 1.19 |
| I | Ile | Isoleucine | 1.38 |

For example, the smaller the normalized hydrophobicity number, the more hydrophilic the amino acid is. For example, the strongly hydrophilic amino acid of the present invention is an amino acid whose normalized hydrophobicity number is less than -0.4, or less than or equal to -0.5, or less than or equal to -0.7, or less than or equal to -0.74. For example, natural "strongly hydrophilic amino acids" include Glu(E), Asn(N), Gln(Q), Asp(D), Lys(K), and Arg(R). The amino acids of the present invention also include artificial amino acids and/or amino acids that modify natural amino acids. Correspondingly, weakly hydrophilic amino acids or hydrophobic amino acids may include amino acids that exhibit a hydrophobicity amount greater than or equal to -0.4, or greater than or equal to -0.5, or greater than or equal to -0.7, or greater than -0.74 on a common hydrophobicity scale, such as His (H), Ser ( S), Thr(T), Pro(P), Ile(I), Phe(F), Val(V), Leu(L), Trp(W), Met(M), Ala(A), Gly( G) and Tyr(Y).

In the present invention, the term "interleukin" generally refers to a cytokine. For example, interleukins can activate and regulate immune cells, mediate the activation, proliferation or differentiation of T cells and B cells, and can play an important role in inflammatory responses. In the present invention, interleukin may encompass unprocessed interleukin, any form of processed interleukin, variants of interleukin, or substances containing functionally active fragments of interleukin.

In the present invention, the term "interleukin-15" or "IL-15" generally refers to a type of cytokine. For example, IL-15 can be found in GenBank accession number P40933. The IL-15 protein of the present invention may also include functionally active fragments thereof, and is not limited to substances containing functionally active fragments of IL-15 produced after processing and/or modification occurring in cells. For example, the IL-15 of the present invention may comprise a functionally active fragment of IL-15 and other arbitrary domains (for example, the extracellular domain, intracellular domain and/or trans- membrane domain). In the present invention, the term "functionally active fragment" or "functional fragment" generally refers to a fragment that has a partial region of a full-length protein or nucleic acid, but retains or partially retains the biological activity or function of the full-length protein or nucleic acid. For example, a functionally active fragment may retain or partially retain the ability of the full-length protein to bind another molecule. For example, the receptor for IL-15 can be IL-15Rα. For example, IL-15Rα can be found in GenBank accession number Q13261. For example, functionally active fragments of IL-15Rα include, but are not limited to, the extracellular domain, intracellular domain, and/or transmembrane domain of IL-15Rα, as well as IL-15Rα-containing functional fragments produced after processing and/or modification that occur in cells. The functionally active fragment of IL-15Rα of the present invention, such as the extracellular domain, may have the function of binding IL-15 or its fragment.

In the present invention, the term "IL-15 structural domain" generally refers to a domain comprising a functionally active structural fragment of IL-15. For example, the IL-15 structural domain of the invention may comprise functionally active fragments of IL-15 and variants thereof. For example, the substance comprising an IL-15 structural domain of the invention may have IL-15's ability of regulating immune cell function.

In the present invention, the term "signal peptide" generally refers to a propeptide present as an N-terminal peptide in the form of a protein precursor. The function of the signal peptide is to promote the translocation of the expressed polypeptide connected to the endoplasmic reticulum and to express the target protein on the cell membrane. The signal peptide can usually be cleaved during this process. For example, the antigen-binding protein of the present invention does not have a signal peptide and does not affect its biological activity. A signal peptide may be heterologous or homologous to the organism used to produce the polypeptide.

In the present invention, the amino acid residues may be naturally occurring amino acids and/or artificially modified amino acids. For example, natural amino acids can be selected from the following group: alanine (three-letter code: ala, one-letter code: A), arginine (arg, R), asparagine (asn, N), aspartic acid ( asp, D), cysteine (cys, C), glutamine (gln, Q), glutamic acid (glu, E), glycine (gly, G), histidine (his, H), iso Leucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y) and valine (val, V).

In the present invention, the term "extracellular domain" generally refers to the extracellular part of the molecule. In the present invention, the term "intracellular domain" generally refers to the intracellular part of the molecule. In the present invention, the term "transmembrane domain" generally refers to the portion that spans the cell membrane. In the present invention, the term "signal peptide domain" generally refers to a short peptide chain that directs the transfer of newly synthesized proteins to the secretory pathway.

In the present invention, the term "immune cell" generally refers to cells involved in immune responses, such as promoting immune effector responses. Examples of immune cells include, but are not limited to, T cells, B cells, natural killer (NK) cells, NKT cells, mast cells, granulocytes, monocytes, lymphocytes, and macrophages. For example, the immune cells of the present invention may include cells derived from artificial pluripotent stem (iPS) cells, PBMC cells, and/or tumor-infiltrating lymphocytes. For example, the immune cells of the present invention can be obtained by differentiation of iPS cells. The term also includes engineered immune cells, such as immune cells that have been genetically modified by adding exogenous genetic material in the form of DNA or RNA to the cell's total genetic material.

In the present invention, the term "killing ability" refers to killing cells by contacting said cells with an effective amount of antibodies, T cells, immunoconjugates, bispecific/multispecific molecules or compositions. For example, the fusion polypeptide of the present invention can enhance the killing ability of cells. For example, cells expressing the fusion polypeptides of the invention may exhibit enhanced killing capabilities. For example, cells expressing the fusion polypeptide of the invention are combined with other immune cells, antibodies, immunoconjugates (e.g., conjugates of binding molecules targeting immune checkpoints and other active molecules), and/or bispecific/multispecific Combinations of sexual molecules can exhibit enhanced killing capabilities. The method may comprise killing cells positive for antigen expression, optionally in the presence of effector cells, for example by CDC, apoptosis, ADCC, phagocytosis or by a combination of two or more of these mechanisms.

In the present invention, the term "direct or indirect connection" refers to a direct connection through a peptide bond, or an indirect connection through a linker peptide or a non-peptide connection.

In the present invention, the term "PBMC" or "human peripheral blood mononuclear cells" generally refers to cells with a single nucleus in peripheral blood. For example, any blood cell with a round nucleus (i.e., lymphocyte, monocyte, or macrophage). These blood cells are key components of the immune system's ability to fight infections and adapt to invaders. Lymphocyte population comprises CD4 + and CD8 + T cells, B cells and natural killer cells, CD14 + monocytes and basophils/neutrophils/eosinophils/dendritic cells. Typically, using FICOLL^{™} (a hydrophilic polysaccharide that stratifies blood), these cells are isolated from whole blood, with monocytes and lymphocytes forming the buffy coat beneath the plasma layer. For example, "PBMC" refers to a cell population that includes at least T cells, and optionally NK cells, NKT cells, and antigen-presenting cells.

In the present invention, the term "proliferation" refers to an increase in cell division (symmetric or asymmetric division of cells). "Proliferation" can refer to symmetric or asymmetric division of T cells. "Increased proliferation" occurs when there is an increase in the number of cells in a treated sample compared to cells in an untreated sample.

In the present invention, the term "subject" includes any human or non-human animal. The term "inhuman animal" includes all vertebrates, such as mammals and non-mammals, such as non-human primates, sheep, dogs, cats, cattle, horses, chickens, amphibians, and reptiles, and may be mammals, such as non-human primates, cats, sheep, dogs, cats, cows and horses.

In the present invention, the term "therapeutically effective amount" refers to an amount of a substance of the invention sufficient to prevent or slow down symptoms associated with a disease or condition (e.g., cancer). The therapeutically effective amount is related to the disease being treated, and those skilled in the art can easily determine the actual effective amount.

In the present invention, the term "drug" generally refers to a chemical compound or composition capable of inducing a desired therapeutic effect when properly administered to a patient.

In the present invention, the term "composition" means a mixture containing one or more compounds according to the invention or physiologically/pharmaceutically acceptable salts or prodrugs thereof and other chemical components, as well as other physiological components such as /Pharmaceutically acceptable carriers and excipients. The purpose of pharmaceutical compositions is to facilitate administration to living organisms and facilitate the absorption of active ingredients to exert biological activity. Therapeutic compositions should generally be sterile and stable under the conditions of manufacture and storage.

In the present invention, the term "vector" generally refers to a nucleic acid molecule capable of transporting another nucleic acid to which it is linked. One type of vector is a "plasmid," which refers to a circular double-stranded DNA circle into which other DNA segments can be ligated. Another type of vector is a viral vector, in which other DNA segments can be ligated into the viral genome. Certain vectors are capable of autonomous replication in the host cells into which they are introduced (e.g., bacterial vectors with bacterial origins of replication and episomal mammalian vectors). Other vectors (e.g., non-episomal mammalian vectors) can be integrated into the host cell's genome when introduced into the host cell, thereby replicating together with the host genome, such as naked RNA polynucleotides, naked DNA polynucleotides, or naked DNA polynucleotides that cannot replicate autonomously. Polynucleotides composed of DNA and RNA in chains, poly-lysine-conjugated DNA or RNA, peptide-conjugated DNA or RNA, liposome-conjugated DNA, etc. In addition, certain vectors are capable of directing the expression of genes to which they are operably linked. This type of vector is called "recombinant expression vector" (or simply "expression vector") in the present invention. Generally speaking, expression vectors used in recombinant DNA technology are usually in the form of plasmids. In this specification, "plasmid" and "vector" are used interchangeably because plasmids are the most commonly used vectors form.

In the present invention, the term "adjuvant" generally refers to any substance that assists or modulates the action of a drug, including but not limited to immunological adjuvants, which enhance or diversify the immune response to an antigen.

In the present invention, the term "tumor" or "tumor cell" generally refers to or describes a physiological condition in mammals that is often characterized by unregulated cell growth. Examples of tumors include, but are not limited to, carcinomas, lymphomas, blastomas (including medulloblastoma and retinoblastoma), sarcomas (including liposarcoma and synovial cell sarcoma), neuroendocrine tumors (including carcinoid tumors , gastrinoma and islet cell carcinoma), mesothelioma, schwannoma (including acoustic neuroma), meningiomas, adenocarcinoma and melanoma. "Tumor cells" may further include "solid tumors" which refer to tumors selected from the group consisting of gastrointestinal cancer, pancreatic cancer, glioblastoma, cervical cancer, ovarian cancer, bladder cancer, hepatoma , breast cancer, colon cancer, rectal cancer, colorectal cancer, endometrial or uterine cancer, salivary gland cancer, kidney cancer, prostate cancer, vulvar cancer, thyroid cancer, liver cancer (hepatic carcinoma), anal cancer, penile cancer, testicular cancer , esophageal cancer, bile duct tumors, and head and neck cancer.

In the present invention, the term "STAT" generally refers to a signal transducer and activator of transcription. For example, STATs of the present invention may comprise STATSA and STAT5B. In the present invention, the term "STAT5A" generally refers to a STAT protein. For example, STAT5A can be found in UniProt accession number P42229. The STAT5A protein of the present invention may also include functionally active fragments thereof, and is not limited to substances containing functionally active fragments of STATSA produced after processing and/or modification occurring in cells. For example, the STAT5A of the present invention may include functionally active fragments of STATSA and other arbitrary structural domains.

In the present invention, the term "Siglec family protein member" generally refers to one or more proteins of the sialic acid-binding immunoglobulin-like lectin family. For example, members of the Siglec family protein members Siglec1 to 7. In the present invention, the term "Siglec15" generally refers to a member of the Siglec family protein. For example, Siglec15 can be found in UniProt accession number Q6ZMC9. The Siglec15 protein of the invention may also include functionally active fragments thereof, and is not limited to substances containing functionally active fragments of Siglec15 produced after processing and/or modification occurring in cells. For example, Siglec15 of the present invention may include a functionally active fragment of Siglec15 and other arbitrary domains (for example, the V-type Ig-like domain, C2-type Ig-like domain, and glycosylation linking domain described in UniProt accession number Q6ZMC9) .

In the present invention, the term "IL-21 receptor" generally refers to the receptor for IL-21 protein. For example, the IL-21 receptor may comprise IL-21Rβ. For example, IL-21Rβ can be found in UniProt accession number Q9HBE5. The IL-21Rβ protein of the present invention may also include functionally active fragments thereof, and is not limited to substances containing functionally active fragments of IL-21Rβ produced after processing and/or modification that occurs in cells. For example, the IL-21Rβ of the present invention may comprise a functionally active fragment of IL-21Rβ and any other structural domain (for example, the intracellular domain, transmembrane domain or intracellular domain of IL-21Rβ described in UniProt registration number Q9HBE5).

In the present invention, the term "CD3ζ" generally refers to CD247 or T-cell surface glycoprotein CD3 zeta chain. CD3ζ" can be found under UniProt accession number P20963. This term includes variants, homologues, and functionally active fragments thereof (e.g., the ITAM domain of CD3ζ described in UniProt accession number P20963).

### Detailed Description

### Mutated interleukin-15 fusion protein with increased membrane anchorage ratio

In one aspect, the invention provides a fusion polypeptide comprising a mutated interleukin-15 (IL-15) receptor or a functional fragment thereof, wherein the percentage of membrane-bound expression product of the fusion polypeptide comprising the mutated interleukin-15 receptor is increased in a cell after intracellular expression, comparing to a protein comprising a native interleukin-15 receptor.

In one aspect, the invention provides a fusion polypeptide comprising a mutated interleukin family member or a functionally active fragment thereof, wherein the percentage of membrane-bound expression product of the fusion polypeptide comprising the mutated interleukin family member is increased in a cell after intracellular expression, comparing to a protein comprising a native interleukin family.

In one aspect, the invention provides a fusion polypeptide comprising a mutated interleukin family member receptor or a functionally active fragment thereof, wherein the percentage of membrane-bound expression product of the fusion polypeptide comprising the mutated interleukin family member receptor is increased in a cell after intracellular expression, comparing to a protein comprising a native interleukin family receptor.

For example, the interleukin family member is selected from: IL-2, IL-7, IL-12, IL-15, and IL-21. For example, the interleukin family member receptor is selected from: IL-2 receptor, IL-7 receptor, IL-12 receptor, IL-15 receptor, and IL-21 receptor. For example, the fusion polypeptide can comprise any interleukin family member linked to any interleukin family member receptor.

For example, compared to a protein containing a naturally occurring or native interleukin-15 receptor, the ratio of membrane-bound expression product of the fusion polypeptide of the present invention can be increased by at least about 1% to about 100 times, for example, by about 1%, by about 5%, increase by about 10%, increase by about 20%, increase by about 30%, increase by about 40%, increase by about 50%, increase by about 70%, increase by about 100%, or can be increased by at least about 1 times, about 1.5 times, or about 2 times , about 5 times, about 10 times, about 20 times, about 50 times, or about 100 times. The proportion of membrane-bound expression product can be determined by methods disclosed in the art, including but not limited to cell flow cytometry.

For example, compared to the protein containing the natural interleukin-15 receptor, the proportion of the expression product of the fusion polypeptide of the present invention that is secreted out of the cell can be reduced by at least about 1% to about 100 times, for example, by about 1%, by about 5 %, reduced by about 10%, reduced by about 20%, reduced by about 30%, reduced by about 40%, reduced by about 50%, reduced by about 70%, reduced by about 100%, or can be reduced by at least about 1 times, about 1.5 times, about 2 times, about 5 times, about 10 times, about 20 times, about 50 times, or about 100 times. The secretion ratio can be determined by methods disclosed in the art, including but not limited to detecting cell culture supernatant through a cytokine detection kit.

For example, the fusion polypeptide of the present invention comparises a mutated interleukin-15 (IL-15) receptor or a functionally active fragment thereof, and the mutated interleukin-15 receptor includes a mutated IL-15 receptor alpha subunit (IL-15Rα ). Compared to the native IL-15 receptor, the mutated transmembrane domain of the alpha subunit comparises at least one amino acid mutation. For example, the fusion polypeptides of the invention comprise the transmembrane domain of the IL-15 receptor alpha subunit having from 1 to 4, such as 1, 2, 3, 4 or more amino acid mutations. For example, the transmembrane domain of the IL-15 receptor α subunit contained in the fusion polypeptide of the present invention comprises amino acid mutations, which can improve the ratio of membrane-bound fusion protein of the present invention, while maintaining or improving the functions of the fusion protein of the present invention.

For example, relative to the transmembrane domain of the native IL-15 receptor α subunit VAISTSTVLLCGLSAVSLLACYL (SEQ ID NO: 17), the mutated transmembrane domain of the present invention may have at least one amino acid mutation in positions 3 to 6 thereof. For example, the mutation may be any 1, 2, 3, 4 or more amino acid mutations in positions 3 to 6 of ISTS. For example, the mutation may be the substitution, insertion, deletion or duplication of an existing amino acid residue by one or more amino acid residues. For example, the mutation can increase the membrane anchoring ratio of the fusion protein of the present invention, while maintaining or improving the activity of the fusion protein of the present invention. For example, the mutation may be a substitution mutation. For example, the mutation may be an insertion mutation. For example, the mutation may be a deletion mutation. For example, the mutation may be a repeat mutation.

For example, relative to the transmembrane domain of the native IL-15 receptor α subunit VAISTSTVLLCGLSAVSLLACYL (SEQ ID NO: 17), the mutated transmembrane domain of the present invention may have at least one amino acid deletion mutation in the 3rd amino acid therein. For example, relative to the transmembrane domain of the native IL-15 receptor α subunit VAISTSTVLLCGLSAVSLLACYL (SEQ ID NO: 17), the mutated transmembrane domain of the present invention may have at least one amino acid deletion mutation in the 4th amino acid therein. For example, relative to the transmembrane domain of the native IL-15 receptor α subunit VAISTSTVLLCGLSAVSLLACYL (SEQ ID NO: 17), the mutated transmembrane domain of the present invention may have at least one amino acid deletion mutation in the 5th amino acid therein. For example, relative to the transmembrane domain of the native IL-15 receptor α subunit VAISTSTVLLCGLSAVSLLACYL (SEQ ID NO: 17), the mutated transmembrane domain of the present invention may have at least one amino acid deletion mutation in the 6th amino acid therein. For example, the mutated interleukin-15 receptor transmembrane domain comprises a sequence selected from any one of SEQ ID NOs: 1-4

For example, the fusion polypeptide can comprise an IL-15 structural domain. For example, the IL-15 domain may be full-length IL-15 or a functionally active fragment thereof. For example, the IL-15 domain may comprise the amino acid sequence shown in SEQ ID NO: 11.

For example, the fusion polypeptide of the present invention can be membrane-bound IL-15 (mbIL-15), such as comprising an IL-15 domain and an IL-15 receptor or a functionally active fragment thereof. For example, the fusion polypeptide may also comprise an IL-15 receptor or a functionally active fragment thereof. For example, the IL-15 receptor may comprise IL-15Rα. For example, the IL-15 receptor of the present invention may comprise the full length of the IL-15 receptor or a functionally active fragment thereof. For example, the IL-15 receptor of the invention may comprise the sushi domain, hinge domain, transmembrane domain and/or intracellular domain of the IL-15 receptor.

For example, the fusion polypeptide may also comprise the extracellular domain of IL-15Rα. For example, the extracellular domain of IL-15Rα may comprise the amino acid sequence shown in SEQ ID NO: 13.

For example, the fusion polypeptide may also comprise the intracellular domain of IL-15Rα. For example, the intracellular domain of IL-15Rα may comprise the amino acid sequence shown in SEQ ID NO: 15.

For example, the IL-15 structural domain and the IL-15 receptor or functionally active fragment thereof may be directly or indirectly linked. For example, the indirect connection may comprise linkage via a linker peptide. For example, the linker peptide may comprise polypeptides known in the art for linking two moieties to be linked. For example, the linker peptide may comprise an amino acid sequence selected from SEQ ID NO:7, SEQ ID NO:8, and SEQ ID NO: 12.

For example, the fusion polypeptide may also include a signal peptide. For example, the signal peptide may comprise an amino acid sequence selected from SEQ ID NO: 5, SEQ ID NO: 6, and SEQ ID NO: 10.

For example, the fusion polypeptide may comprise the IL-15 structural domain shown in SEQ ID NO: 11, the linker peptide shown in any one of SEQ ID NO: 7, SEQ ID NO: 8, and SEQ ID NO: 12, and the transmembrane IL-15Rα shown in any one of SEQ ID NOs: 1-4.

For example, the fusion polypeptide may comprise the IL-15 structural domain shown in SEQ ID NO: 11, the linker peptide shown in any one of SEQ ID NO: 7, SEQ ID NO: 8, and SEQ ID NO: 12, the extracellular domain of IL-15Rα shown in SEQ ID NO: 13, and the transmembrane domain of IL-15Rα shown in any one of SEQ ID NOs: 1-4.

For example, the fusion polypeptide may comprise the signal peptide shown in any one of SEQ ID NO: 5, SEQ ID NO: 6, and SEQ ID NO: 10, the IL-15 structural domain shown in SEQ ID NO: 11, The linker peptide shown in any one of SEQ ID NO: 7, SEQ ID NO: 8, and SEQ ID NO: 12, the extracellular domain of IL-15Rα shown in SEQ ID NO: 13, the transmembrane domain of IL-15Rα shown in any one of SEQ ID Nos: 1-4, and the intracellular domain of IL-15Rα shown in SEQ ID NO: 15.

For example, the fusion polypeptide comprises the amino acid sequence shown in SEQ ID NO: 9. For example, the sequences mentioned in the present invention include sequences that are at least about 95%, at least about 96%, at least about 97%, at least about 98%, and at least about 99% identical to the sequence.

For example, the fusion polypeptide comprises the amino acid sequence shown in SEQ ID NO: 18. For example, the sequences mentioned in the present invention include sequences that are at least about 95% identical to the sequence, at least about 96%, at least about 97%, at least about 98%, and at least about 99% identical to the sequence.

For example, the fusion polypeptide comprises the amino acid sequence shown in SEQ ID NO: 19. For example, the sequences mentioned in the present invention include sequences that are at least about 95% identical to the sequence, at least about 96%, at least about 97%, at least about 98%, and at least about 99% identical to the sequence.

For example, the fusion polypeptide comprises the amino acid sequence shown in SEQ ID NO:20. For example, the sequences mentioned in the present invention include sequences that are at least about 95% identical to the sequence, at least about 96%, at least about 97%, at least about 98%, and at least about 99% identical to the sequence.

For example, the fusion polypeptide sequence mentioned in the present invention comprises a sequence that is at least about 80% identical to the sequence of SEQ ID NO: 9, 18, 19, or 20 (except for the fusion of wild-type IL-15 and wild-type IL-15Rα). For example, the sequences described in the present invention include sequences that are at least about 85% identical to the sequence of SEQ ID NO: 9, 18, 19, or 20 (except for the fusion protein of wild-type IL-15 and wild-type IL-15Rα). ). For example, the fusion polypeptide sequence described in the present invention includes a sequence that is at least about 90% identical to the sequence of SEQ ID NO: 9, 18, 19, or 20 (except for the fusion of wild-type IL-15 and wild-type IL-15Rα).

For example, the sequence described in the present invention includes at least about 80%, at least about 85%, at least about 90%, at least about 95%, at least about 96%, at least about 97%, at least about 98%, at least about 99% % identical sequences.

For example, the fusion polypeptide may also contain additional intracellular signaling domains. For example, the intracellular signaling domain is selected from the following group: STAT family protein members or functionally active fragments thereof, Siglec family protein members or functionally active fragments thereof, IL-21 receptors or functionally active fragments thereof, and CD3ζ or functionally active fragments thereof .

For example, the STAT family protein member may comprise STAT5A. For example, the STAT family protein member may comprise STATS. For example, the STAT family protein member may comprise STAT5A, a variant thereof, or a functionally active fragment thereof. For example, the STAT5A may comprise a mutation at amino acid position 298 and/or a mutation at amino acid 710. For example, the STAT5A may comprise a mutation of amino acid 298 to R (arginine) and/or a mutation of amino acid 710 to F (phenylalanine).

For example, the Siglec family protein member may comprise Siglec15. For example, the Siglec family protein member may comprise Siglec15, a variant thereof, or a functionally active fragment thereof. For example, the fusion polypeptide may also comprise the transmembrane domain of Siglec15. For example, the fusion polypeptide may also comprise the intracellular domain of Siglec15.

For example, the IL-21 receptor may comprise IL-21Rβ. For example, the IL-21 receptor of the present invention may comprise the full length of the IL-21 receptor or a functionally active fragment thereof. For example, the fusion polypeptide may also comprise the intracellular domain of 4-1BB. For example, the fusion polypeptide may also comprise an activation motif of a receptor tyrosine, such as the signaling domain of CD3ζ.

In one aspect, the invention provides a nucleic acid encoding a fusion polypeptide of the invention.

In one aspect, the invention provides a vector that may comprise a nucleic acid of the invention.

In one aspect, the invention provides a cell that may comprise the fusion polypeptide of the invention, the nucleic acid of the invention and/or the vector of the invention.

For example, the cells may comprise immune cells. For example, the cells may comprise NK cells, NKT cells and/or T cells. For example, the cells may comprise cells derived from induced pluripotent stem (iPS) cells, PBMC cells, and/or tumor-infiltrating lymphocytes. For example, the cells of the present invention can be obtained by differentiation of iPS cells.

In one aspect, the present invention provides a method for preparing the fusion polypeptide of the present invention, comprising culturing the cells of the present invention under conditions that allow the expression of the fusion polypeptide.

In one aspect, the invention provides a composition, which may comprise a fusion polypeptide of the invention, a nucleic acid of the invention, a vector of the invention, and/or a cell of the invention, and optionally a pharmaceutically acceptable carrier.

In one aspect, the invention provides a kit, which may comprise the fusion polypeptide of the invention, the nucleic acid of the invention, the vector of the invention, the cell of the invention, and/or the composition of the invention.

In one aspect, the invention provides a method for culturing cells, which may comprise providing the fusion polypeptide of the invention, the nucleic acid of the invention, the vector of the invention, the cell of the invention, the composition of the invention, and/or the kit of the present invention. For example, the method of culturing cells of the present invention may be to transduce a vector expressing the fusion polypeptide of the present invention into the cells.

In one aspect, the invention provides the use of the fusion polypeptide of the invention, the nucleic acid of the invention, the vector of the invention, the cell of the invention, the composition of the invention and/or the kit of the invention in the preparation of medicines in a medicament to prevent, alleviate, and/or treat tumors.

In one aspect, the invention provides the fusion polypeptide of the invention, the nucleic acid of the invention, the vector of the invention, the cell of the invention, the composition of the invention and/or the kit of the invention for use in preventing, alleviating and /or treating tumors.

In one aspect, the present invention provides a method for preventing, alleviating and/or treating tumors, the method comprising providing the fusion polypeptide of the present invention, the nucleic acid of the present invention, the vector of the present invention, the cell of the present invention, and the composition of the present invention materials and/or kits of the present invention.

### Interleukin-15 fusion proteins containing hydrophilic linkers or linker peptides

### Embodiments

A linker peptide, comprising a number of strongly hydrophilic amino acids that accounts for 15% to 40% of the total number of amino acids, and the strongly hydrophilic amino acids are selected from Glu(E), Asn(N), Gln(Q), Asp (D), Lys(K) and Arg(R).

The linker peptide according to the linker peptide of embodiment 1, containing a total number of amino acids of 18 to 35.

The linker peptide according to any one of embodiments 1-2, comprising the amino acid sequence shown in any one of SEQ ID NOs: 26-48.

A fusion protein comprising the linker peptide described in any one of embodiments 1-3.

The fusion protein according to embodiment 4, which includes the structure of K1-L-K2, wherein L is the linker peptide, K1 includes the first polypeptide, and K2 includes the second polypeptide.

The fusion protein according to embodiment 5, wherein K1 and K2 are each independently an interleukin or a functional fragment thereof.

The fusion protein according to any one of embodiments 5-6, wherein the K1 comprises interleukin-15 (IL-15) or a functional fragment thereof.

The fusion protein according to embodiment 7, wherein the IL-15 or functional fragment thereof comprises the amino acid sequence shown in SEQ ID NO: 24.

The fusion protein according to any one of embodiments 5-8, wherein the K2 comprises interleukin-15 receptor alpha (IL-15Rα) or a functional fragment thereof.

The fusion protein according to any one of embodiments 5-9, wherein the K2 comprises the extracellular domain of IL-15Rα.

The fusion protein according to embodiment 10, wherein the extracellular domain of IL-15Rα comprises the amino acid sequence shown in SEQ ID NO: 49.

The fusion protein according to any one of embodiments 5-11, wherein the K2 comprises the intracellular domain of IL-15Rα.

The fusion protein according to embodiment 12, wherein the intracellular domain of IL-15Rα comprises the amino acid sequence shown in SEQ ID NO: 56.

The fusion protein according to any one of embodiments 5-13, wherein the K2 comprises the transmembrane domain of IL-15Rα.

The fusion protein according to embodiment 14, wherein the transmembrane domain of IL-15Rα comprises the amino acid sequence shown in any one of SEQ ID NO: 50-55.

The fusion protein according to any one of embodiments 5-15, further comprising a signal peptide located at the N-terminus of K1.

The fusion protein according to embodiment 16, wherein the signal peptide comprises the amino acid sequence shown in any one of SEQ ID NO: 21-23.

The fusion protein according to any one of embodiments 4-17, comprising the amino acid sequence shown in any one of SEQ ID NO: 57-62.

A method for preparing the fusion protein according to any one of embodiments 4-18, which includes linking two or more fragments of the fusion protein through the linker peptide according to any one of embodiments 1-3.

A nucleic acid encoding the linker peptide described in any one of embodiments 1-3 and/or the fusion protein described in any one of embodiments 4-18.

A vector comprising the nucleic acid according to embodiment 20.

A cell comprising the linker peptide described in any one of embodiments 1-3, the fusion protein described in any one of embodiments 4-18, the nucleic acid described in Embodiment 20, and/or the carrier of embodiment 21.

The cell according to embodiment 22, comprising immune cells.

The cell according to any one of embodiments 22-23, said cell comprising NK cells, NKT cells and/or T cells.

The cell according to any one of embodiments 22-24, comprising cells derived from induced pluripotent stem cells (iPSCs), PBMC cells and/or tumor-infiltrating lymphocytes.

A composition comprising the linker peptide described in any one of embodiments 1-3, the fusion protein described in any one of embodiments 4-18, the nucleic acid described in embodiment 20, the nucleic acid described in embodiment 21, the cell of any one of embodiments 22-25, and/or the composition of described in embodiment 26, and optionally a pharmaceutically acceptable carrier.

A kit comprising the linker peptide described in any one of embodiments 1-3, the fusion protein described in any one of embodiments 4-18, the nucleic acid described in embodiment 20, the vector described in embodiment 21, the cell described in any one of embodiments 22-25, and/or the composition described in embodiment 26.

A method for culturing cells, comprising providing the linker peptide described in any one of embodiments 1-3, the fusion protein described in any one of embodiments 4-18, the nucleic acid described in embodiment 20, the vector described in embodiment 21, the cell according to any one of embodiments 22-25, the composition according to embodiment 26, and/or the kit according to embodiment 27.

A linker peptide according to any one of embodiments 1 to 3, a fusion protein according to any one of embodiments 4 to 18, a nucleic acid according to embodiment 20, a vector according to embodiment 21, use of the cell according to any one of 22-25, the composition according to embodiment 26 and/or the kit according to embodiment 27 in the preparation of a medicament for prevention, alleviation and/or treatment of diseases (preferably tumors).

A medicament for preventing and/or treating diseases and/or symptoms (preferably tumors), comprising the linker peptide described in any one of embodiments 1-3, the fusion protein described in any one of embodiments 4-18, the nucleic acid of embodiment 20, the vector of embodiment 21, the cell of any one of embodiments 22-25, the composition of embodiment 26, and/or the kit of embodiment 27.

A method for preventing and/or treating diseases and/or symptoms (preferably tumors), comprising administering to a subject in need the linker peptide described in any one of embodiments 1-3, the fusion protein described in any one of embodiments 4-18, the nucleic acid described in embodiment 20, the vector described in embodiment 21, the cell described in any one of embodiments 22-25, the composition described in embodiment 26, and/or the kit described in embodiment 27.

The linker peptide described in any one of embodiments 1-3, the fusion protein described in any one of embodiments 4-18, the nucleic acid described in embodiment 20, the vector described in embodiment 21, the cell described in any one of embodiments 22-25, the composition according to embodiment 26 and/or the kit according to embodiment 27 for use in preventing and/or treating diseases and/or symptoms (preferably tumors).

### linker peptide

In one aspect, the invention provides a linker peptide. For example, the linker peptide of the invention may be hydrophilic. For example, the linker peptide of the present invention is more hydrophilic than the GGS linker peptide. For example, the hydrophilic character of the linker peptide of the invention can be determined by the ratio of strongly hydrophilic amino acids therein. For example, strongly hydrophilic amino acids include amino acids with a normalized hydrophobicity number of less than -0.4. For example, strongly hydrophilic amino acids include Glu(E), Asn(N), Gln(Q), Asp(D), Lys(K), and Arg(R).

For example, the number of strongly hydrophilic amino acids contained in the linker peptide of the present invention accounts for 15% to 40% of the total number of all amino acids in the linker peptide. For example, the number of strongly hydrophilic amino acids included in the linker peptide of the present invention accounts for about 15% to 40%, about 20% to 40%, about 25% to 40%, and about 30% of the total number of all amino acids in the linker peptide. to 40%, about 35% to 40%, about 15% to 35%, about 20% to 35%, about 25% to 35%, about 30% to 35%, about 15% to 30%, about 15% to 30%, about 16% to 30%, about 18% to 30%, about 20% to 30%, about 20% to 30%, about 21% to 30%, about 23% to 30%, about 25% to 30 %, about 27% to 30%, about 29% to 30%, about 15% to 27%, about 16% to 27%, about 18% to 27%, about 20% to 27%, about 20% to 27% , about 21% to 27%, about 23% to 27%, about 25% to 27%, about 15% to 23%, about 16% to 23%, about 18% to 23%, about 20% to 23%, about 20% to 23%, about 21% to 23%, about 15% to 20%, about 16% to 20%, about 18% to 20%, about 15% to 18%, about 16% to 18%. For example, the number of strongly hydrophilic amino acids contained in the linker peptide of the present invention accounts for about 15% or more, about 17% or more, about 19% or more, about 20% or more, or about 21% or more of the total number of all amino acids in the linker peptide. , about 23% or more, about 25% or more, about 27% or more, about 29% or more, about 30% or more, about 35% or more, or about 40% or more. For example, the ratio of the number of strongly hydrophilic amino acids contained in the linker peptide of the present invention to the total number of all amino acids in the linker peptide is about 15%, about 16%, about 17%, about 18%, about 19%, about 20%, About 21%, about 22%, about 23%, about 24%, about 25%, about 26%, about 27%, about 28%, about 29%, about 30%, about 31%, about 32%, about 33 %, about 34%, about 35%, about 36%, about 37%, about 38%, about 39%, or about 40%. For example, the number of strongly hydrophilic amino acids contained in the linker peptide of the present invention accounts for 19.23% to 36.00% of the total number of all amino acids in the linker peptide.

For example, the linker peptide of the present invention includes about 18 to 35 amino acids. For example, the linker peptide of the present invention includes a total of about 18, about 19, about 20, about 21, about 22, about 23, about 24, about 25, about 26, about 27, about 28, about 29, about 30, about 31, about 32, about 33, about 34 or about 35 amino acids.

For example, the linker peptide of the present invention includes the amino acid sequence shown in any one of SEQ ID NOs: 26-48. For example, the sequences referred to in the present invention include sequences that are at least about 80% identical to the sequence.

For example, the sequence described in the present invention contains at least about 80%, at least about 85%, at least about 90%, at least about 95%, at least about 96% of the sequence, at least about 97% of the sequence, at least about 98%, a sequence that is at least about 99% identical to the sequence. Preferably, the linker peptide includes the amino acid sequence of SEQ ID NOs: 26-48 after conservative substitution.

For example, the linker peptide can be used to link two or more components, such as molecules or peptides.

For example, the linker peptide can be used to link two or more non-peptide molecules. For example, the linker peptide can be used to link two or more peptide molecules. For example, the linker peptide can be used to connect different types of components, for example, one or some components are non-peptide small molecule substances, and the other or some components are peptides. For example, the substance connected through the linker peptide in the present invention can be called a fusion protein or a fusion polypeptide (the two terms are used interchangeably in the present invention).

### Fusion Protein

In another aspect, the present invention provides a fusion protein comprising the hydrophilic linker peptide of the present invention.

For example, the fusion protein of the present invention includes the structure of K1-L-K2, where L is the linker peptide, K1 includes the first polypeptide, and K2 includes the second polypeptide. For example, the first and second polypeptides of the invention may comprise molecules known in the art, such as cytokines, growth factors, hormones, enzymes, receptors, ligands, antibodies, and the like. For example, L may comprise the amino acid sequence shown in any one of SEQ ID NOs: 26-48. For example, L therein may comprise the amino acid sequence shown in any one of SEQ ID NO: 27 and 46. For example, L therein may comprise the amino acid sequence shown in any one of SEQ ID NOs: 27, 29, 39, 43 and 47.

For example, the linker peptide of the present invention is used to connect any interleukin family member to any interleukin family member receptor. For example, the interleukin family member is selected from: IL-2, IL-7, IL-12, IL-15, and IL-21. For example, the interleukin family member receptor is selected from the group consisting of: IL-2 receptor, IL-7 receptor, IL-12 receptor, IL-15 receptor, and IL-21 receptor. For example, the fusion polypeptide can be composed of any interleukin family member linked to any interleukin family member receptor.

For example, K1 and K2 in the fusion protein of the present invention are each independently interleukin or a functional fragment thereof.

For example, the K1 in the fusion protein of the present invention includes interleukin-15 (IL-15) or its functional fragments. For example, the fusion polypeptide can comprise an IL-15 domain. For example, the IL-15 domain may be full-length IL-15 or a functionally active fragment thereof.

For example, the IL-15 or functional fragment thereof in the fusion protein of the present invention includes the amino acid sequence shown in SEQ ID NO: 24.

For example, the K2 in the fusion protein of the present invention includes interleukin-15 receptor alpha (IL-15Rα) or a functional fragment thereof. For example, the fusion polypeptide of the invention may comprise an IL-15 structural domain and an IL-15 receptor or a functionally active fragment thereof. For example, the fusion polypeptide may also comprise an IL-15 receptor or a functionally active fragment thereof. For example, the IL-15 receptor may comprise IL-15Rα. For example, the IL-15 receptor of the present invention may comprise the full length of the IL-15 receptor or a functionally active fragment thereof. For example, the IL-15 receptor of the present invention may comprise the sushi domain (sushi domain, also known as the sushi domain), hinge domain, transmembrane domain and/or intracellular domain of the IL-15 receptor.

For example, the K2 in the fusion protein of the present invention includes the extracellular domain of IL-15Rα. For example, the extracellular domain of IL-15Rα in the fusion protein of the present invention includes the amino acid sequence shown in SEQ ID NO: 49.

For example, the K2 in the fusion protein of the present invention contains the intracellular domain of IL-15Rα. For example, the intracellular domain of IL-15Rα in the fusion protein of the present invention includes the amino acid sequence shown in SEQ ID NO: 56.

For example, the K2 in the fusion protein of the present invention includes the transmembrane domain of IL-15Rα. For example, the transmembrane domain of IL-15Rα in the fusion protein of the present invention includes the amino acid sequence shown in any one of SEQ ID NOs: 50-55.

For example, the fusion protein of the present invention also contains a signal peptide located at the N-terminus of K1. For example, the signal peptide in the fusion protein of the present invention includes the amino acid sequence shown in any one of SEQ ID NOs: 21-23.

For example, the fusion protein of the present invention includes the amino acid sequence shown in any one of SEQ ID NOs: 57-62. For example, the fusion protein mentioned in the present invention contains a sequence that is at least about 80% identical to any one of SEQ ID NOs: 57-62 (except for wild-type IL-15 and wild-type IL-15Rα by a linker peptide only containing G and S). For example, the fusion protein mentioned in the present invention contains a sequence that is at least about 85% identical to any one of SEQ ID NO: 57-62 (except for wild-type IL-15 and wild-type IL-15Rα by a linker peptide only containing G and S). For example, the fusion protein mentioned in the present invention contains a sequence that is at least about 90% identical to any one of SEQ ID NO: 57-62 (except for wild-type IL-15 and wild-type IL-15Rα by a linker peptide only containing G and S). For example, a sequence referred to in the present invention includes a sequence that is at least about 95% identical to that sequence. For example, the sequence mentioned in the present invention contains at least about 80%, at least about 85%, at least about 90%, at least about 95%, at least about 96% of the sequence, at least about 97% of the sequence, at least about 98%, a sequence that is at least about 99% homologous to the sequence. Preferably, the fusion protein of the present invention includes the amino acid sequence of one of SEQ ID NOs: 57-62 after conservative substitution.

For example, the fusion protein of the present invention includes IL-15 as shown in SEQ ID NO:24 or a functional fragment thereof, a linker peptide as shown in any one of SEQ ID NO:26-48, an extracellular domain of IL-15Rα as shown in SEQ ID NO:49. For example, the fusion protein of the present invention includes IL-15 as shown in SEQ ID NO:24 or a functional fragment thereof, a linker peptide as shown in any one of SEQ ID NOs:27-46, an extracellular domain of IL-15Rα as shown in SEQ ID NO:49. For example, the fusion protein of the present invention includes IL-15 or a functional fragment thereof as shown in SEQ ID NO:24, a linker peptide as shown in any one of SEQ ID NOs:27, 29, 39, 43 and 47, the extracellular domain of IL-15Rα as shown in SEQ ID NO:49.

For example, the fusion polypeptide may also contain additional intracellular signaling domains. For example, the intracellular signaling domain is selected from the following group: STAT family protein members or functionally active fragments thereof, Siglec family protein members or functionally active fragments thereof, IL-21 receptors or functionally active fragments thereof, and CD3ζ or functionally active fragments thereof .

For example, the STAT family protein member may comprise STAT5A. For example, the STAT family protein member may comprise STATS. For example, the STAT family protein member may comprise STAT5A, a variant thereof, or a functionally active fragment thereof. For example, the STAT5A may comprise a mutation at amino acid 298 and/or a mutation at amino acid 710. For example, the STAT5A may comprise a mutation of amino acid 298 to R (arginine) and/or a mutation of amino acid 710 to F (phenylalanine).

For example, the Siglec family protein member may comprise Siglec15. For example, the Siglec family protein member may comprise Siglec15, a variant thereof, or a functionally active fragment thereof. For example, the fusion polypeptide may also comprise the transmembrane domain of Siglec15. For example, the fusion polypeptide may also comprise the intracellular domain of Siglec15.

For example, the IL-21 receptor may comprise IL-21Rβ. For example, the IL-21 receptor of the present invention may comprise the full length of the IL-21 receptor or a functionally active fragment thereof. For example, the fusion polypeptide may also comprise the intracellular domain of 4-1BB. For example, the fusion polypeptide may also comprise an activation motif of a receptor tyrosine, such as the signaling domain of CD3ζ.

For example, the fusion polypeptide of the invention may also comprise a mutated interleukin-15 (IL-15) receptor or a functionally active fragment thereof, the mutated interleukin-15 receptor comprising a mutated transmembrane domain of IL-15 receptor alpha subunit (IL-15) or a functionally active fragment thereof, the mutated transmembrane domain having at least one amino acid mutation relative to the transmembrane domain of the natural IL-15 receptor α subunit. For example, the fusion polypeptides of the invention comprise the transmembrane domain of the IL-15 receptor alpha subunit having one or more, for example 1, 2, 3, 4 or more amino acid mutations. For example, the transmembrane domain of the IL-15 receptor α subunit contained in the fusion polypeptide of the present invention has amino acid mutations, which can improve the ratio of the membrane-bound fusion protein of the present invention, while maintaining or improving the functions of the fusion protein of the present invention.

For example, relative to the transmembrane domain of the native IL-15 receptor α subunit, the mutated transmembrane domain of the present invention may have any one or more of the four ISTS amino acids at positions 3 to 6, such as 1, 2, 3, 4 or more amino acid mutations. For example, the mutation can be the substitution, insertion, deletion or duplication of an existing amino acid residue by one or more amino acid residues. For example, the mutation can increase the membrane anchoring ratio of the fusion protein of the present invention, while maintaining or improving the activity of the fusion protein of the present invention. For example, the mutation may be a substitution mutation. For example, the mutation may be an insertion mutation. For example, the mutation may be a deletion mutation. For example, the mutation may be a repeat mutation.

In one aspect, the present invention provides a method for preparing the fusion protein of the present invention, which includes connecting two or more fragments of the fusion protein through the linker peptide of the present invention.

In one aspect, the invention provides the use of the linker peptide of the invention in preparing fusion proteins.

In one aspect, the invention provides a nucleic acid encoding a fusion polypeptide of the invention.

In one aspect, the invention provides a vector that may comprise a nucleic acid of the invention.

In one aspect, the invention provides a cell, which may comprise the fusion protein of the invention, the nucleic acid of the invention and/or the vector of the invention.

For example, the cells may comprise immune cells. For example, the cells may comprise NK cells, NKT cells and/or T cells. For example, the cells may comprise cells derived from induced pluripotent stem (iPS) cells, PBMC cells, and/or tumor-infiltrating lymphocytes. For example, the cells of the present invention can be obtained by differentiation of iPS cells.

In one aspect, the present invention provides a method for preparing the fusion protein of the present invention, which method includes culturing the cells of the present invention under conditions that allow the expression of the fusion protein.

In one aspect, the invention provides a composition which may comprise the fusion protein, the nucleic acid of the invention, the vector of the invention, and/or the cell of the invention, and optionally a pharmaceutically acceptable carrier.

In one aspect, the invention provides a kit, which may comprise the fusion protein of the invention, the nucleic acid of the invention, the vector of the invention, the cell of the invention, and/or the composition of the invention.

In one aspect, the invention provides a method for culturing cells, which may include providing the fusion protein of the invention, the nucleic acid of the invention, the vector of the invention, the cells of the invention, the composition of the invention, and/or the kit of the present invention. For example, the method of culturing cells of the present invention may be to transduce a vector expressing the fusion protein of the present invention into the cells.

In one aspect, the present invention provides the use of the fusion protein of the present invention, the nucleic acid of the present invention, the vector of the present invention, the cell of the present invention, the composition of the present invention and/or the kit of the present invention in the preparation of a medicament to prevent, alleviate, and/or treat disease. For example, the disease includes tumors.

In one aspect, the invention provides the fusion protein of the invention, the nucleic acid of the invention, the vector of the invention, the cell of the invention, the composition of the invention and/or the kit of the invention for use in preventing, alleviating and /or treating diseases. For example, the disease includes tumors.

In one aspect, the present invention provides a method for preventing, alleviating and/or treating diseases, the method comprising providing the fusion protein of the present invention, the nucleic acid of the present invention, the vector of the present invention, the cell of the present invention, and the composition of the present invention and/or kits of the present invention. For example, the disease includes tumors.

Without intending to be bound by any theory, the following examples are merely to illustrate the products, preparation methods and uses of the present invention, and are not intended to limit the scope of the present invention.

### Examples

Sequence information of fusion proteins

| SEQ ID NO: | Name | Sequence |
|---|---|---|
| 1 | D-S181 | VAISTTVLLCGLSAVSLLACYL |
| 2 | D-I178 | VASTSTVLLCGLSAVSLLACYL |
| 3 | D-S179 | VAITSTVLLCGLSAVSLLACYL |
| 4 | D-T180 | VAISSTVLLCGLSAVSLLACYL |
| 5 | SP1 | MRISKPHLRSISIQCYLCLLLNSHFLTEA |
| 6 | SP2 | MVAGSDAGRALGVLSVVCLLHCFGFISC |
| 7 | Linker A | SGGGSGGGGSGGGGSGGGGSGGGGSGGGSLQ |
| 8 | Linker B | SGGGSGGGGSGGGGSGGGGSGGGGSGGGGSGGGSLQ |
| 9 | LS-1 | MDWTWILFLVAAATRVHSNWVNVISDLKKIEDLIQSMHIDATLYTESDV HPSCKVTAMKCFLLELQVISLESGDASIHDTVENLIILANNSLSSNGNVTES GCKECEELEEKNIKEFLQSFVHIVQMFINTSSGGGSGGGGSGGGGSGGGG SGGGSLQITCPPPMSVEHADIWVKSYSLYSRERYICNSGFKRKAGTSSLTE CVLNKATNVAHWTTPSLKCIRDPALVHQRPAPPSTVTTAGVTPQPESLSP SGKEPAASSPSSNNTAATTAAIVPGSQLMPSKSPSTGTTEISSHESSHGTPS QTTAKNWELTASASHQPPGVYPQGHSDTTVAISTTVLLCGLSAVSLLACY LKSRQTPPLASVEMEAMEALPVTWGTSSRDEDLENCSHHL |
| 10 | SP | MDWTWILFLVAAATRVHS |
| 11 | IL-15 | NWVNVISDLKKIEDLIQSMHIDATLYTESDVHPSCKVTAMKCFLLELQVIS LESGDASIHDTVENLIILANNSLSSNGNVTESGCKECEELEEKNIKEFLQSF VHIVQMFINTS |
| 12 | Linker | SGGGSGGGGSGGGGSGGGGSGGGSLQ |
| 13 | IL-15RαEX | ITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAAS SPSSNNTAATTAAIVPGSQLMPSKSPSTGTTEISSHESSHGTPSQTTAKNW ELTASASHQPPGVYPQGHSDTT |
| 14 | IL-15RαTM | VA[I or missing][S or missing][T or missing][S or missing]TVLLCGLSAVSLLACYL |
| 15 | IL-15RαCY | KSRQTPPLASVEMEAMEALPVTWGTSSRDEDLENCSHHL |
| 16 | HS | |
| 17 | IL-15RαTM (HS) | VAISTSTVLLCGLSAVSLLACYL |
| 18 | LS-2 | MDWTWILFLVAAATRVHSNWVNVISDLKKIEDLIQSMHIDATLYTESDV HPSCKVTAMKCFLLELQVISLESGDASIHDTVENLIILANNSLSSNGNVTES GCKECEELEEKNIKEFLQSFVHIVQMFINTSSGGGSGGGGSGGGGSGGGG SGGGSLQITCPPPMSVEHADIWVKSYSLYSRERYICNSGFKRKAGTSSLTE CVLNKATNVAHWTTPSLKCIRDPALVHQRPAPPSTVTTAGVTPQPESLSP SGKEPAASSPSSNNTAATTAAIVPGSQLMPSKSPSTGTTEISSHESSHGTPS QTTAKNWELTASASHQPPGVYPQGHSDTTVASTSTVLLCGLSAVSLLAC YLKSRQTPPLASVEMEAMEALPVTWGTSSRDEDLENCSHHL |
| 19 | LS-3 | MDWTWILFLVAAATRVHSNWVNVISDLKKIEDLIQSMHIDATLYTESDV HPSCKVTAMKCFLLELQVISLESGDASIHDTVENLIILANNSLSSNGNVTES GCKECEELEEKNIKEFLQSFVHIVQMFINTSSGGGSGGGGSGGGGSGGGG SGGGSLQITCPPPMSVEHADIWVKSYSLYSRERYICNSGFKRKAGTSSLTE CVLNKATNVAHWTTPSLKCIRDPALVHQRPAPPSTVTTAGVTPQPESLSP SGKEPAASSPSSNNTAATTAAIVPGSQLMPSKSPSTGTTEISSHESSHGTPS QTTAKNWELTASASHQPPGVYPQGHSDTTVAITSTVLLCGLSAVSLLACY LKSRQTPPLASVEMEAMEALPVTWGTSSRDEDLENCSHHL |
| 20 | LS-4 | MDWTWILFLVAAATRVHSNWVNVISDLKKIEDLIQSMHIDATLYTESDV HPSCKVTAMKCFLLELQVISLESGDASIHDTVENLIILANNSLSSNGNVTES GCKECEELEEKNIKEFLQSFVHIVQMFINTSSGGGSGGGGSGGGGSGGGG SGGGSLQITCPPPMSVEHADIWVKSYSLYSRERYICNSGFKRKAGTSSLTE CVLNKATNVAHWTTPSLKCIRDPALVHQRPAPPSTVTTAGVTPQPESLSP SGKEPAASSPSSNNTAATTAAIVPGSQLMPSKSPSTGTTEISSHESSHGTPS QTTAKNWELTASASHQPPGVYPQGHSDTTVAISSTVLLCGLSAVSLLACY LKSRQTPPLASVEMEAMEALPVTWGTSSRDEDLENCSHHL |
| 21 | Signal peptide | MDWTWILFLVAAATRVHS |
| 22 | Signal peptide 1 | MRISKPHLRSISIQCYLCLLLNSHFLTEA |
| 23 | Signal peptide 2 | MVAGSDAGRALGVLSVVCLLHCFGFISC |
| 24 | IL-15 | NWVNVISDLKKIEDLIQSMHIDATLYTESDVHPSCKVTAMKCFLLELQVIS LESGDASIHDTVENLIILANNSLSSNGNVTESGCKECEELEEKNIKEFLQSF VHIVQMFINTS |
| 25 | Linker Control | SGGGSGGGGSGGGGSGGGGSGGGSLQ |
| 26 | Linker1 | KESGSVSSEQLAQFRSLDSGSESKST |
| 27 | Linker2 | KESGSVSSEQLAQFRSLDSAAGSGEF |
| 28 | Linker3 | GEDSTAPGEDSTAPGSAGSAAGSGEF |
| 29 | Linker4 | GQAPKESGSVSSEQLAQFRSLDGQAP |
| 30 | Linker5 | SSEQLAQFRSLDEGKSSGSGSESKST |
| 31 | Linker6 | SGSESKSTKESGSVSSEQLAQFRSLD |
| 32 | Linker7 | SAAGSGEFKESGSVSSEQLAQFRSLD |
| 33 | Linker8 | GEDSTAPGQAPGQAPGQAPGEDSTAP |
| 34 | Linker9 | KESGSVSSEQLAQFRSLDGEDSTAPG |
| 35 | Linker10 | GEDSTAPGSAGSAAGSGEFGEDSTAP |
| 36 | Linker11 | GQAPGQAPKESGSVSSEQLAQFRSLD |
| 37 | Linker12 | GQAPKESGSVSSEQLAQFRSLDSGGS |
| 38 | Linker13 | GQAPGPASGEDSTAPGEDSTAPGQAP |
| 39 | Linker14 | KESGSVSSEQLAQFRSLDGEDSTAP |
| 40 | Linker15 | KESGSVSSEQLAQFRSLDEGKSSGSGSESKST |
| 41 | Linker16 | KESGSVSSEQLAQFRSLDGSAGSAAGSGEF |
| 42 | Linker17 | KESGSVSSEQLAQFRSLDGQAP |
| 43 | Linker18 | EGKSSGSGSESKSTGSAGSAAGSGEF |
| 44 | Linker19 | EGKSSGSGSESKSTGQAPGSAGSAAGSGEF |
| 45 | Linker20 | EGKSSGSGSESKSTGEDSTAPGQAP |
| 46 | Linker21 | EGKSSGSGSESKSTPASGQAPPAS |
| 47 | Linker22 | EGKSSGSGSESKSTPASGEDSTAP |
| 48 | Linker23 | KESGSVSSEQLAQFRSLDPAS |
| 49 | IL-15RαEX | ITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAAS SPSSNNTAATTAAIVPGSQLMPSKSPSTGTTEISSHESSHGTPSQTTAKNW ELTASASHQPPGVYPQGHSDTT |
| 50 | IL-15RαTM | VA[I or missing] [S or missing] [T or missing] [S or missing]TVLLCGLSAVSLLACYL |
| 51 | IL-15RαTM 1 | VAISTTVLLCGLSAVSLLACYL |
| 52 | IL-15RαTM 2 | VASTSTVLLCGLSAVSLLACYL |
| 53 | IL-15RαTM 3 | VAITSTVLLCGLSAVSLLACYL |
| 54 | IL-15RαTM 4 | VAISSTVLLCGLSAVSLLACYL |
| 55 | IL-15RαTM 5 | VAISTSTVLLCGLSAVSLLACYL |
| 56 | IL-15RαCY | KSRQTPPLASVEMEAMEALPVTWGTSSRDEDLENCSHHL |
| 57 | PC163 | |
| 58 | PC228 | MDWTWILFLVAAATRVHSNWVNVISDLKKIEDLIQSMHIDATLYTESDV HPSCKVTAMKCFLLELQVISLESGDASIHDTVENLIILANNSLSSNGNVTES GCKECEELEEKNIKEFLQSFVHIVQMFINTSSGGGSGGGGSGGGGSGGGG SGGGSLQITCPPPMSVEHADIWVKSYSLYSRERYICNSGFKRKAGTSSLTE CVLNKATNVAHWTTPSLKCIRDPALVHQRPAPPSTVTTAGVTPQPESLSP SGKEPAASSPSSNNTAATTAAIVPGSQLMPSKSPSTGTTEISSHESSHGTPS QTTAKNWELTASASHQPPGVYPQGHSDTTVAISTTVLLCGLSAVSLLACY LKSRQTPPLASVEMEAMEALPVTWGTSSRDEDLENCSHHL |
| 59 | PC241 | MRISKPHLRSISIQCYLCLLLNSHFLTEANWVNVISDLKKIEDLIQSMHIDA TLYTESDVHPSCKVTAMKCFLLELQVISLESGDASIHDTVENLIILANNSLS SNGNVTESGCKECEELEEKNIKEFLQSFVHIVQMFINTSKESGSVSSEQLA QFRSLDSAAGSGEFITCPPPMSVEHADIWVKSYSLYSRERYICNSGFKRKA GTSSLTECVLNKATNVAHWTTPSLKCIRDPALVHQRPAPPSTVTTAGVTP QPESLSPSGKEPAASSPSSNNTAATTAAIVPGSQLMPSKSPSTGTTEISSHES SHGTPSQTTAKNWELTASASHQPPGVYPQGHSDTTVAISTTVLLCGLSAV SLLACYLKSRQTPPLASVEMEAMEALPVTWGTSSRDEDLENCSHHL |
| 60 | PC242 | MVAGSDAGRALGVLSVVCLLHCFGFISCNWVNVISDLKKIEDLIQSMHID ATLYTESDVHPSCKVTAMKCFLLELQVISLESGDASIHDTVENLIILANNS LSSNGNVTESGCKECEELEEKNIKEFLQSFVHIVQMFINTSKESGSVSSEQL AQFRSLDSAAGSGEFITCPPPMSVEHADIWVKSYSLYSRERYICNSGFKRK AGTSSLTECVLNKATNVAHWTTPSLKCIRDPALVHQRPAPPSTVTTAGVT PQPESLSPSGKEPAASSPSSNNTAATTAAIVPGSQLMPSKSPSTGTTEISSHE SSHGTPSQTTAKNWELTASASHQPPGVYPQGHSDTTVAISTTVLLCGLSA VSLLACYLKSRQTPPLASVEMEAMEALPVTWGTSSRDEDLENCSHHL |
| 61 | PC243 | MRISKPHLRSISIQCYLCLLLNSHFLTEANWVNVISDLKKIEDLIQSMHIDA TLYTESDVHPSCKVTAMKCFLLELQVISLESGDASIHDTVENLIILANNSLS SNGNVTESGCKECEELEEKNIKEFLQSFVHIVQMFINTSGQAPKESGSVSS EQLAQFRSLDGQAPITCPPPMSVEHADIWVKSYSLYSRERYICNSGFKRK AGTSSLTECVLNKATNVAHWTTPSLKCIRDPALVHQRPAPPSTVTTAGVT PQPESLSPSGKEPAASSPSSNNTAATTAAIVPGSQLMPSKSPSTGTTEISSHE SSHGTPSQTTAKNWELTASASHQPPGVYPQGHSDTTVAISTTVLLCGLSA VSLLACYLKSRQTPPLASVEMEAMEALPVTWGTSSRDEDLENCSHHL |
| 62 | PC244 | MVAGSDAGRALGVLSVVCLLHCFGFISCNWVNVISDLKKIEDLIQSMHID ATLYTESDVHPSCKVTAMKCFLLELQVISLESGDASIHDTVENLIILANNS LSSNGNVTESGCKECEELEEKNIKEFLQSFVHIVQMFINTSGQAPKESGSV SSEQLAQFRSLDGQAPITCPPPMSVEHADIWVKSYSLYSRERYICNSGFKR KAGTSSLTECVLNKATNVAHWTTPSLKCIRDPALVHQRPAPPSTVTTAGV TPQPESLSPSGKEPAASSPSSNNTAATTAAIVPGSQLMPSKSPSTGTTEISSH ESSHGTPSQTTAKNWELTASASHQPPGVYPQGHSDTTVAISTTVLLCGLS AVSLLACYLKSRQTPPLASVEMEAMEALPVTWGTSSRDEDLENCSHHL |

### Example 1. Cell sources and cell culture

Cells expressing the fusion protein of the present invention can be any cells, including but not limited to immune cells. For example, immune cells can be derived from tumor samples of clinical patients, and the tumor blocks are ground, digested, and sorted to obtain immune cells, such as TILs. Immune cells were stimulated with CD3 antibodies and activated in commercial immune cell culture media for one to three days before transduction.

### Plasmid construction

The plasmid construction of the retrovirus carrying the fusion polypeptide can be accomplished by synthesizing the gene fragment encoding the fusion polypeptide of the present invention, digesting it with EcoRI+NotI, and recovering the exogenous fragment fusion polypeptide. Plasmid MP71 was digested with EcoRI+NotI and the vector fragment was recovered. Use T4Ligase to connect the foreign fragment and the vector fragment to obtain a retrovirus plasmid carrying the fusion polypeptide.

For example, the combination of natural IL-15 and IL-15Rα is used to construct the mbIL-15 fusion protein sequence as shown in SEQ ID NO: 16, which can be used as the control group HS (high secretion). The combination of IL-15 and IL-15Rα provided by the present invention comprising a mutated transmembrane domain (such as the mutated sequence shown in any one of SEQ ID NO: 1 to 4) is used to construct mbIL-15 fusion protein, for example, the sequence can LS-1 as shown in SEQ ID NO: 9 can be used as the test group LS (low secretion).

### Cell transduction

One day before transduction, a 24-well suspension culture plate was coated with recombinant human fibrin fragment (Retronectin, Takara) at a final concentration of 10-20 µg/mL, 250 µL per well of the 24-well plate. The plate was protected from light and stored overnight at 4°C for later use. The coated 24-well plate was then taken out, the coating solution discarded, and 500 µL of blocking solution containing 2% BSA was added for blocking at room temperature for 30 minutes. The blocking solution was aspirated and discarded. The plate was washed twice with 500 µL/well of washing solution containing 2.5% HEPES, and the washing solution was discarded. The test group was transduced with a retrovirus carrying the nucleic acid sequence encoding the fusion protein of the present invention.

0.25-2mL retrovirus solution was added to each well, centrifuged at 32°C, 1000-2000g for 1-2 hours, and no cell transduction was performed in the negative control group. The supernatant from the 24-well plate was discarded and immune cells were added, such as TIL group, to each well of the 24-well plate with a volume of 300-500 µL to reach a cell concentration of approximately 0.5-2×10 6 pieces/mL. The culture plate was placed at 37°C, 5% CO₂ and cultured in an incubator to obtain transduced cells. 1-3 days after transduction, the cells were resuspended and counted, and trophoblast cells were added according to the cell number TIL:trophoblast cells=1:40~1:400. 3-6 days after transduction, fresh culture medium containing IL-2 with a concentration of 300-9000IU/mL (for example, 1000-9000IU/mL) was supplemented in a timely manner depending on the growth status of the cells. Thereafter, the cells were observed every 2-4 days, and cultured with timely replenishment to maintain the cell concentration at 1E6-2E6/mL.

### Example 2. Transduction efficiency

Starting from the 7th day after transduction, a flow cytometer (Beckman Cytoflex) could be used to detect the transduction efficiency of the immune cells of the present invention.

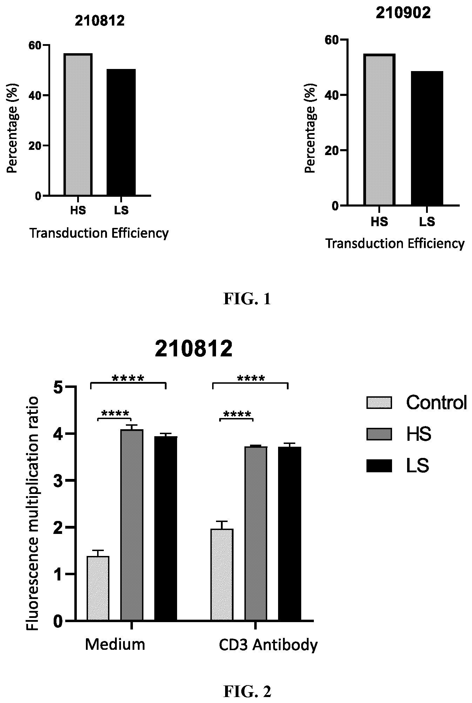

FIG. 1 shows the mbIL-15 transduction efficiency of T cells in each group. Among different TIL donors, the control group HS (high secretion) and the test group LS (low secretion, for example, the sequence can be LS-1 as shown in SEQ ID NO: 9) have similar expression efficiencies on TIL cells after transduction. The results show that the mutation expression provided by the invention did not affect the expression efficiency of the fusion protein in cells.

### Promotion of cell proliferation

Beginning on the 7th day after transduction, 30ng/mL anti-CD3 antibody was plated as the CD3 antibody group, and no anti-CD3 antibody was plated as the unstimulated group (i.e. culture medium group). The next day, T cells in the transduction group and control group were plated in a 96-well plate at 1.5e5/well, and 3 days later, the amplification was detected. According to the instructions of the CTG kit (CellTiter-Glo Luminescent Cell Viability Assay, Promega), the CTG reaction solution was prepared by mixing the CTG substrate (CellTiter-Glo Substrate) and the CTG buffer (CellTiter-Glo Buffer). The detection cell suspension was added into a 96-well microplate, 50 µL/well, and the well containing only culture medium was set as the background value of fluorescence. An equal volume of CTG reaction solution was added to each well, shaken on a horizontal shaker for 2 minutes, and let stand in the dark at room temperature for 10 minutes to stabilize the fluorescence signal, and then the fluorescence value was read. The above method was used to detect cell proliferation ability.

FIG. 2 shows the proliferation ability of T cells in each group. Relative to the significance of the Control group of TCR-T cells not transduced with mbIL-15 fusion protein, **** indicates p<0.0001. The results show that the immune cells transduced with the fusion polypeptide of the control group HS or the test group LS (low secretion, for example, the sequence could be as shown in SEQ ID NO: 9) fusion polypeptide have significantly enhanced expansion ability, and the degree of enhancement was similar.

### Promotion of cytokine secretion

On the 7th day after transduction, 30ng/mL anti-CD3 antibody was plated as the CD3 antibody group, and no anti-CD3 antibody was plated as the unstimulated group (i.e., culture medium group). The next day, T cells from the transduction group and the control group were Plate 1.5e5/well into a 96-well plate, and the supernatant was taken after 24 hours for cytokine secretion detection.

For the cytokine secretion detection method, the instructions of the cytokine detection kit (BD) was referred to. The human Th1/Th2/Th17 cytokine standard freeze-dried powder (BD) was reconstituted with 2 mL Assay Diluent diluent (BD) (the concentration of each cytokine in the standard solution was 5000pg/mL) and was serially diluted 1:2, 1:4, 1:8, 1:16, 1:32, 1:64, 1:128, 1:256, 1:512, 1:1024, labeled "standard tube". One tube containing only Assay Diluent was used as a reference. Each Capture Beads (BD) was added at 2µL/Beads/well, then PE Detection Reagent (BD) was added at 10µL/well and mixed to prepare a mixture, which was added at 22µL/well into the V-bottom 96-well plate. The supernatant of each standard and experimental group was added at 10 µL/well, mixed, and incubated at room temperature in the dark for 3 hours.

At the end of the incubation, 200 µL Wash Buffer (BD) was added to each well and centrifuged at 500g for 3 minutes. After centrifugation, 100 µL Wash Buffer (BD) was added to each well to resuspend and analyzed by flow cytometry.

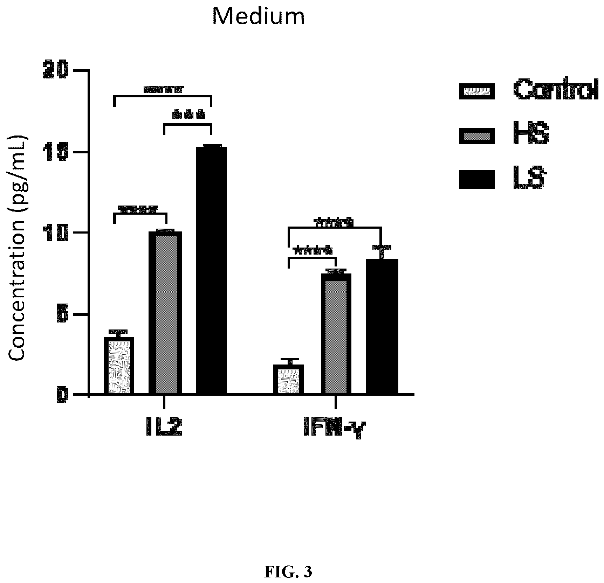

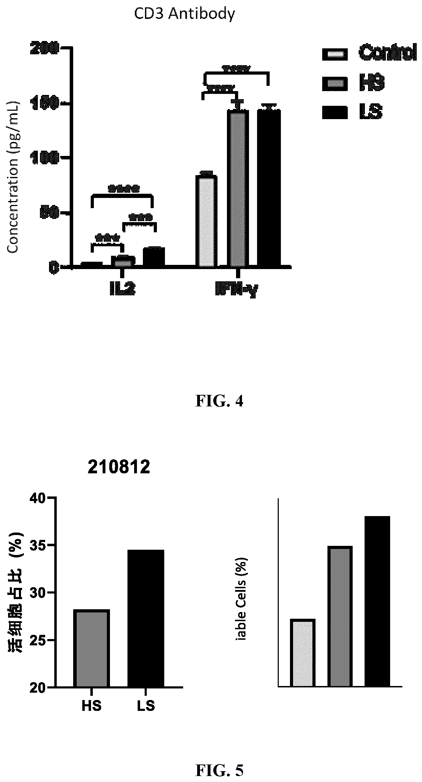

FIGs. 3 and 4 show the secretion of cytokines by T cells in the medium group and CD3 antibody group. Relative to the significance of the TCR-T cell Control group or the control HS group without transduction of mbIL-15 fusion protein, *** represents p<0.001, and **** represents p<0.0001. The test group was LS (low secretion, for example, the sequence could be LS-1 as shown in SEQ ID NO: 9).

The results showed that the immune cells transduced with HS in the control group or the LS fusion polypeptide of the present invention could have significantly enhanced cytokine secretion ability, and the ability of LS to promote IL-2 secretion was stronger than that of the HS group.

### Cell conditions

Starting from the 7th day after transduction, a flow cytometer (Beckman Cytoflex) and Live/Dead detection reagent could be used to detect the status of the immune cells of the present invention. The higher the proportion of viable cell population, the better the cell status was. TCR-T cells that were not transduced with mbIL-15 fusion protein were used as the Control group.

FIG. 5 shows the proportion of viable cells of T cells in each group. The cell viability of the immune cells transduced with the LS (low secretion, for example, the sequence could be as shown in SEQ ID NO: 9) fusion polypeptide of the test group of the present invention was higher in both TIL donors than in the control group HS, indicating that LS could better improve the viability and status of TIL than HS.

### Promotion of the continuous killing of tumor cells

Beginning on the 8th day after transduction, A375 tumor cell lines were cultured at 2 × 10⁴/well in a 96-well plate. After 2 hours, TIL cells in each group were co-cultured with the tumor cells at an effect-to-target ratio (TIL cells: tumor cells) of 4:1. Each group had at least three wells, and Caspase3/7 substrate was added to the wells, which were placed into IncuCyte for scanning. Three days later, the A375 tumor cell line was re-cultured at 2 × 10⁴ /well in a 96-well plate, and TILs were collected from the co-culture system, centrifuged, resuspended, counted, and co-cultured with tumor cells at a 4:1 ratio.

Cells were co-cultured with at least three wells in each group, Caspase3/7 substrate was added to the wells, and IncuCyte was used for scanning; thereafter, the above operation was repeated every three days. TCR-T cells that were not transduced with mbIL-15 fusion protein were used as the Control group.

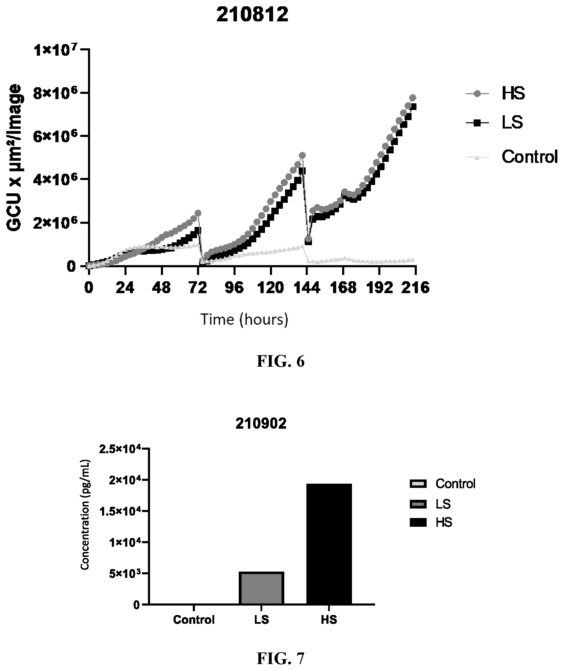

FIG. 6 shows the continuous killing of tumor cells by T cells in each group. The results showed that the immune cells transduced with the fusion polypeptide of the control group HS and the test group LS of the present invention (low secretion, for example, the sequence could be as shown in SEQ ID NO: 9) could have significantly enhanced continuous killing of tumor cells. Enhancements were not affected.

### Detection of IL-15 secretion in supernatant

On the 8th day after transduction, 3E6 cells/well were cultured continuously for 4 days in 3mL #5 medium (without IL-2), the supernatant was taken, and IL-15 complex concentration was detected by ELISA. For the detection method, the instructions of the Human IL-15/IL-15R alpha Complex DuoSet ELISA detection kit (R&D) was referred to. The standard was reconstituted with 0.5mL Reagent Diluent (original standard concentration was 4000pg/mL) and serially diluted 1:2, 1:4, 1:8, 1:16, 1:32, 1:64, 1:128, labeled "standard tube". One tube containing only Assay Diluent was used as a reference. Capture Ab was diluted with PBS to 4 µg/ml, 100 µl/well was coated on the plate, covered, and incubated overnight at room temperature; 100 µl/well standard, sample, and positive control were added, covered, and incubated at room temperature for 2 hours; the plate was washed 3 times with Wash buffer; 100 µl/well detection antibody working solution was added, covered, and incubated for 2 hours; 100 µl/well Streptavidin-HRP working solution (1:200 dilution) was added, covered, and incubated for 20 minutes; 100 µl/well substrate working solution was added and developed in the dark for 10-20 minutes; 50 µl/well stop solution was added and mixed gently; detection was performed at 450 nm. TCR-T cells that were not transduced with mbIL-15 fusion protein were used as the Control group.

FIG. 7 shows the secretion of IL-15 by T cells in each group. The results show that the immune cells transduced with the LS (low secretion, for example, the sequence could be as shown in SEQ ID NO: 9) fusion polypeptide of the test group of the present invention had significantly lower IL-15 secretion in the culture supernatant than the control group HS. Secretion was potentially higher when infused back into the body.

### Example 3. In vivo anti-tumor effect and body weight changes

Immunodeficient mice (NOG mice, Viton Lever, strain code 408) were subcutaneously inoculated with A375 cell line, and the inoculation number was 1 × 10⁶ cells/cell. Seven days after inoculation, when the tumor volume grew to approximately 50 mm³, the mice were randomly divided into groups.

TILs in the LS and HS groups were tested by flow cytometry before reinfusion and had similar IL-15 transduction efficiency and cell viability. Mice in the LS group and HS group were reinfused with 0.5-5 × 10⁷ TIL cells, after which approximately 0.1-5 × 10⁵ IU IL-2 was administered as combination therapy; IL-2 was administered intraperitoneally on the day of therapeutic cell injection, twice a day for 3 consecutive days. The tumor volume and mouse body weight of mice in each group were measured twice a week until 18 days after administration.

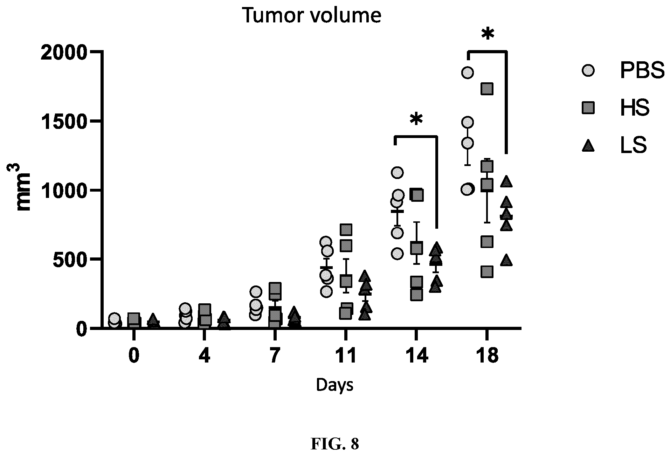

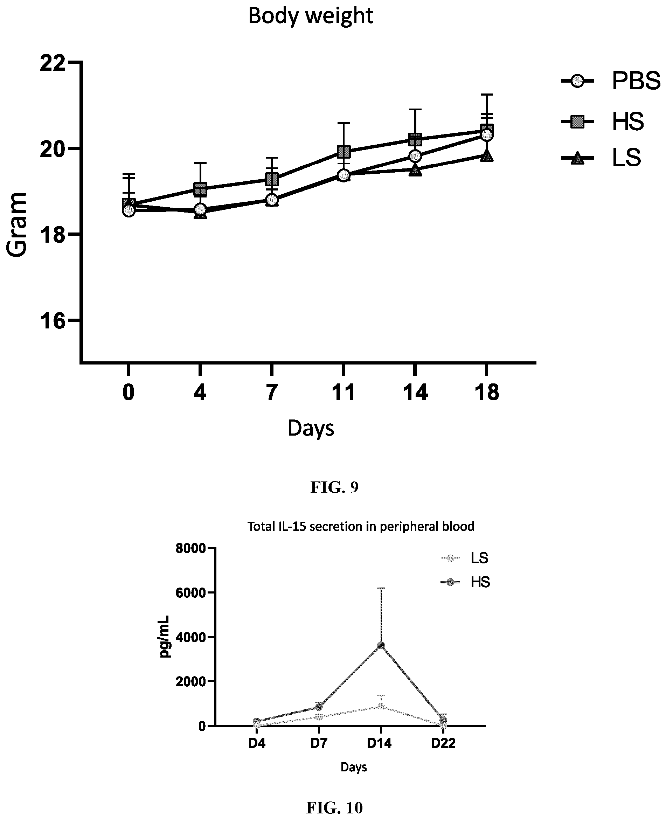

FIGs. 8 and 9 show the tumor volume and body weight of mice in each group. The results show that on days 14 and 18 after administration, the test group LS (low secretion, for example, the sequence could be as shown in SEQ ID NO: 9 LS-1) group significantly inhibited tumor growth compared with the PBS group (*, P<0.05), and body weight had no significant effect; accordingly, the control group HS group did not show a significant tumor inhibition trend compared with the PBS group.

### Detection of IL-15 secretion in peripheral blood

Peripheral blood was collected from mice in each group on Day 4, Day 7, Day 14, and Day 22 after treatment, and plasma was collected by centrifugation. The secretion level of IL-15 in the serum was detected by ELISA.

For the detection method, the instructions of the Human IL-15/IL-15R alpha Complex DuoSet ELISA detection kit (R&D) was referred to. The standard was reconstituted with 0.5mL Reagent Diluent (the original standard solution was 4000pg/mL) and was serially diluted 1:2, 1:4, 1:8, 1:16, 1:32, 1:64, and 1:128, labeled as "standard tubes". One tube containing only Assay Diluent was used as a reference. Capture Ab was diluted with PBS to a working concentration of 4 µg/ml, 100 µl/well was coated on the well plate, covered, and incubated at room temperature overnight; 100 µl/well standard, sample (5-fold dilution), and positive control were added, covered, and incubated at room temperature for 2 hours.

The plate was washed three times with Wash buffer; 100 µl/well detection antibody working solution was added, covered, and incubated at room temperature for 2 hours; 100 µl/well Streptavidin-HRP working solution (1:200 dilution) was added, covered, and incubated at room temperature for 20 minutes; 100 µl/well substrate working solution was added and developed in the dark for 10-20 minutes; 50 µl/well stop solution was added and tapped gently to mix thoroughly; detection was performed at 450 nm.

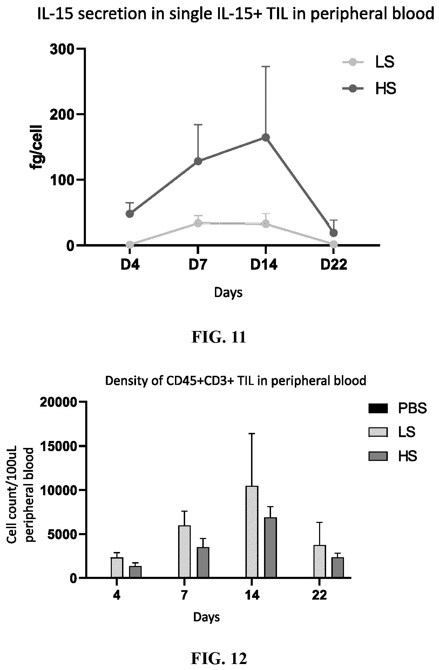

FIGs. 10 and 11 show the secretion of IL-15 in the peripheral blood of T mice in each group. The results showed that on day 14 (D14), the total amount of IL-15 secretion in the serum of mice in the control group HS and the test group LS (low secretion, for example, the sequence could be as shown in SEQ ID NO: 9) reached the peak level, and the total amount of IL-15 secretion in the LS group, or a single TIL cell expressing IL-15 (IL15^{÷} TIL), was lower than that of the HS group, suggesting that the LS group was potentially safer when reinfused into the body.

### Phenotypic analysis of mbIL-15 transduced TIL cells in peripheral blood

Peripheral blood was collected from mice in each group on Day 4, Day 7, Day 14, and Day 22 after T cell reinfusion, and flow cytometry was used to analyze the number and phenotype of T cells in the peripheral blood.

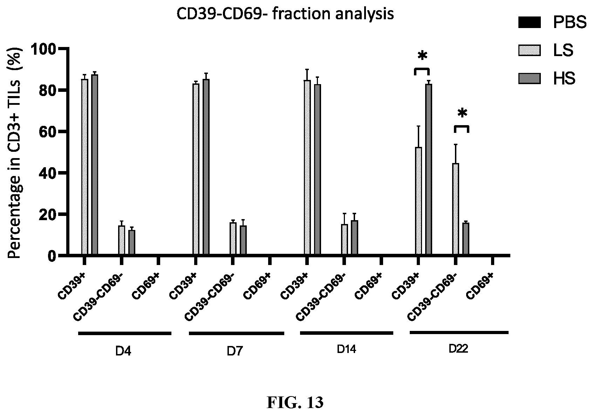

FIGs. 12 and 13 show the number and phenotype of T cells in the peripheral blood of mice in each group. The results show that there was no significant difference in the density of TILs in peripheral blood at each time point after administration. In the test group LS of the present invention (low secretion, for example, the sequence could be as shown in SEQ ID NO: 9), on D22 after reinfusion, CD39⁺ exhausted T cells in the HS group were significantly lower in proportion than in the control group, and the stem subset CD39⁻CD69⁻ T cells were significantly higher in proportion than in the HS group. It is known in the art that the CD39-negative CD69-negative subpopulation is associated with higher stemness and better tumor suppression in patients, suggesting that TIL cell depletion in the LS group was reduced and cell stemness was enhanced after reinfusion compared with the HS group.

### Example 4. Detection of fusion proteins containing various mutated transmembrane domains

### TCR-T cell preparation

T cells from various sources such as peripheral blood and stem cell differentiation, preferably PBMC cells, were taken and cultured. The nucleic acid encoding TCR was transferred into the above-mentioned T cells according to methods known in the art, such as viral transduction, LNP transduction, electroporation, etc. Thus TCR-T cells were obtained.

Before, after or at the same time as transducing TCR into T cells as described above, each experimental group was transduced with a retrovirus carrying nucleic acid encoding mbIL-15 fusion protein to obtain TCR-T cells transduced with IL-15 fusion protein.

TCR-T cells that were not transduced with the mbIL-15 fusion protein were used as a control group, and TCR-T cells that were transduced with the mbIL-15 fusion protein of the wild-type IL-15Rα transmembrane domain were used as a secretion control group (as shown in SEQ ID NO: 16 Indicated, recorded as: HS, high secretion). Cells transduced with the mbIL-15 fusion protein with mutated transmembrane domain were used as the experimental group (for example, those with the sequence shown in SEQ ID NO: 9 are designated as LS-1, those with the sequence shown in SEQ ID NO: 18 were designated as LS-2, The one shown in SEQ ID NO: 19 was designated as LS-3, and the one shown in SEQ ID NO: 20 was designated as LS-4).

The above-obtained TCR-T cells transduced with each group of mbIL-15 fusion protein were used. On the 7th day after transduction, the supernatant ELISA (R&D) was used to detect the IL-15/IL-15Rα complex concentration, and cell counting was performed. The number of viable cells was detected with a flow cytometer (Chemometec), and the expression ratio of IL-15Rα was detected with a flow cytometer (Beckman Coulter).

| Donor | Group | IL-15Rα positive viable cell count | IL15-IL15Ra complex concentration (pg/ml ) | Secretion (fg/ml/cell) |
|---|---|---|---|---|
| 1 | HS | 1.49E+06 | 1468.02 | 0.99 |
| | LS-2 | 1.92E+06 | 1467.545 | 0.76 |
| | LS-3 | 2.45E+06 | 1679.485 | 0.69 |
| | LS-4 | 3.17E+06 | 1062.465 | 0.34 |
| 2 | HS | 2.18E+06 | 2150.74 | 0.99 |
| | LS-2 | 3.43E+06 | 2118.46 | 0.62 |
| | LS-3 | 3.06E+06 | 1916.63 | 0.63 |
| | LS-4 | 2.62E+06 | 1809.405 | 0.69 |

The results are shown in the table above. The IL-15/IL-15Rα fusion protein containing the transmembrane domain mutation of the present invention had a lower extracellular secretion level.

### Example 5. Culture Method of Tumor Infiltrating Lymphocytes (TIL) Cells

### 1.1 Feeder cell reception and preparation

### 1.1.1 Reception of single blood collection

Single blood collection information, batch number and volume were recorded, and rewarmed to room temperature.

### 1.1.2 Manual isolation and cryopreservation of PBMC (peripheral blood mononuclear cells)

Lymphocyte separation solution (Tianjin Haoyang Ficoll) was used to separate the apheresis blood. After the separation, if there were more red blood cells in the buffy coat layer, red blood splitting could be performed after the separation. After the red blood splitting, the cells were washed twice, centrifuged at 400g for 6 minutes, and centrifuged for the last time. Pre-sample count was performed.

The supernatant was discarded, the cells were resuspended in basic medium, and the cell density was adjusted to about 2-3×10⁷ cells/mL; the liquid level height could not exceed 1 cm. In the flat state, X-ray irradiation was performed. The cells were centrifuged, the supernatant was discarded, and the cells were frozen according to the counting results; the cells were placed in a programmed cooling box and transferred to a -80°C refrigerator for freezing.

### 1.1.3 Automatic separation and freezing of PBMC

The blood bag pipeline was aseptically connected to the input end of the cpro separation kit (Cytiva). The neatcell program was used to separate and wash PBMC. The washing solution was physiological saline; the resuspension solution was the basic culture medium. After separation, each donor's PBMC was a bag. In a flat state, the liquid level could be no more than 1 cm and irradiated by X-rays. After irradiation, samples were taken and counted, and cells were collected and washed using the culture wash program; they were frozen in a programmed cooling instrument and stored in liquid nitrogen.

### 1.2 Tumor tissue reception and processing

### 1.2.1 Organizational reception

The donor's tumor tissue and blood samples were received, sample information was checked and recorded, and the corresponding sample labels were printed.

### 1.2.2 Tissue processing and culture

75% alcohol was used to disinfect sample tubes and blood collection tubes, and they were transferred to a biological safety cabinet. PBMC cells were isolated from blood samples and frozen according to the above manual PBMC isolation and cryopreservation procedures. A culture bottle or culture bag with a breathable surface, such as a culture bag (Origen), was taken, and 300 mL of rewarmed complete culture medium was added. The complete culture medium could be arbitrarily selected from X-vivo15 culture medium or other commercial T cell culture media, such as Stem Cell, Lonza, Thermo, Miltenyi, and other brands of T cell culture media, and essential amino acids and antibiotics could be added at a concentration of 300-9000 IU/mL (for example, it could be 1000-9000 IU/mL, such as 6000 IU/mL) of IL-2. Several 10 cm petri dishes were taken, an appropriate amount of culture medium was added, and the tumor tissue was removed from the sample tube and placed in a 10 cm culture dish. The amount of culture medium was based on covering the tumor tissue. The tissue morphology was observed and recorded. The tissue was washed, and the culture dish was replaced. Ophthalmic scissors and ophthalmic tweezers were used to perform preliminary cutting to remove fatty tissue and necrotic tissue, and each tissue block was continued to be minced to approximately 27 cubic millimeters in size. About 1g of non-suspended tumor tissue was transferred into the culture bag through a syringe. The culture bag was placed into a carbon dioxide incubator for culture to obtain the primary TIL population.

### 1.3 The first stage of expansion and harvesting

### 1.3.1 First stage in vitro amplification

Depending on the growth status of the cells, fluid was added or half of the fluid was changed every 3-7 days to ensure cell nutrition. Complete culture medium was used. The complete culture medium could be arbitrarily selected from X-vivo 15 culture medium or other commercial T cell culture media, such as Stem Cell, Lonza, Thermo, Miltenyi, and other brands of T cell culture media, and essential amino acids and antibiotics could be added, along with IL-2 (Double Heron or Quadricycle Bio) at a concentration of 300-9000 IU/mL (for example, it could be 1000-9000 IU/mL, such as 6000 IU/mL). Samples were taken and counted between days 3-14 of the first stage of expansion. If the number of cells was 5 × 10⁵ to 5×10⁸, the harvesting step of the first stage of amplification in 1.3.2 below was entered.

### 1.3.2 Gains from the first stage of amplification

The cells were collected at the end of the first stage of in vitro expansion, centrifuged, the culture medium was discarded, the cells were washed once with PBS or normal saline, and TILs amplified in the first stage of in vitro were obtained. Cells were sampled to enter the second stage of in vitro amplification or quality control detection; the remaining cells were added to cryopreservation solution and frozen as cryopreserved preREP TIL in vitro cells.

### 1.4 Second-stage amplified TIL activation

The TILs expanded in vitro in the first stage of 1.3 were continued to be cultured, or the cryopreserved preREP TIL in vitro cells were thawed to activate the TILs expanded in the second stage.

Complete medium was used. The complete medium could be X-vivo 15 medium or other commercial T cell culture media, such as Stem Cell, Lonza, Thermo, Miltenyi, and other brands of T cell culture media. Essential amino acids and antibiotics were added, and the cell density was adjusted to 5×10⁵ to 2×10⁶ cells/mL. IL-2 was added at a concentration of 300-9000 IU/mL (for example, it could be 1000-9000 IU/mL, such as 6000 IU/mL). Approximately 30 ng/mL CD3 antibody (Miltenyi Biotech, OKT3) and 30 ng/mL CD28 antibody (Merck, 15E8) were added to the culture medium of each TIL cell group simultaneously. Magnetic beads (diameter approximately 1 to 10 µm, Dynabeads, Thermo Fisher) were added at a ratio of TIL and/or transACT (diameter approximately 100 to 500 nm, Miltenyi) was added to TIL at a ratio of approximately 1:100-1:2000. After culturing for about 0-4 days, the activated TIL population was obtained.

### 1.5 TIL cell transduction

The TIL activated in step 1.4 above was transduced. One day before transduction, 24-well suspension culture plates and 24-well plates were coated with recombinant human fibrin fragment (Retronectin, Takara) at a final concentration of 15 µg/mL. The plates were protected from light and stored overnight at 4°C for later use. The coated 24-well plate was taken, the coating solution was discarded, and blocking solution containing 2% BSA was added to block at room temperature for 30 minutes. The blocking solution was aspirated and discarded, the plate was washed twice with a washing solution containing 2.5% HEPES, and the washing solution was discarded. Each test group was transduced with a retrovirus carrying a nucleic acid encoding an IL-15/IL-15Rα fusion protein containing a hydrophilic linker (the amino acid sequence of the hydrophilic linker could be as shown in SEQ ID NO: 26-48 of the present invention).

The plasmid construction method of the above retrovirus was as follows: the IL-15 gene fragment of the present invention was synthesized, digested with EcoRI+NotI, and the exogenous fragment IL-15 was recovered. Plasmid MP71 was digested with EcoRI+NotI, and the vector fragment was recovered. T4Ligase was used to ligate the exogenous fragment and the vector fragment to obtain the retrovirus plasmid carrying IL-15.

0.25-2 mL retroviral solution was added to each well, and centrifuged at 32°C, 2000g for 2 hours. The non-transduced control group did not undergo cell transduction, or the GS linker control group used control virus for transduction. The supernatant from the 24-well plate was discarded, and the TIL population activated in step 1.4 above was added to each well of the 24-well plate, with a volume of 300-500 µL and a cell concentration of approximately 1×10⁶ cells/mL. The plate was centrifuged at 30-32°C, 1000g, for 10 minutes. After centrifugation, the culture plate was placed at 37°C, 5% CO₂ incubator for about 0-4 days, and the post-transduction TIL population was obtained. The obtained TILs were named PC163, PC228, PC241, PC242, PC243, and PC244 (the IL-15/IL-15Rα fusion protein sequences included in each group were SEQ ID NO: 57-62, respectively). The fusion protein shown in SEQ ID NO: 57-58 contained GS linker as the control group; the fusion protein shown in SEQ ID NO: 59-60 contained linker2 shown in SEQ ID NO: 27 as the test group; and the fusion protein shown in SEQ ID NO: 61-62 included linker4 shown in SEQ ID NO: 29 as the test group.

### 1.6 Expanded culture of the second stage expansion of TIL cells after transduction

The feeder cells prepared in step 1.1 above were added to the transduced TIL cell population obtained in step 1.5 for culture. Contact of TIL with feeder cells could occur simultaneously or sequentially with contact of TIL with IL-2 and T cell activators. For example, 0 hours to 12 days after the TIL was exposed to IL-2 and a T cell activator (such as a CD3 antibody or a nanomatrix containing a CD3 antibody and a CD28 antibody, such as transACT), such as at the same time, 24 hours, or 48 hours, the feeder cells could be added to the TIL medium. The feeder cells mixed from 1-5 donors were resuscitated; the activated TIL cells and feeder cells were mixed at a ratio of TIL cells:feeder cells of about 1:50 to 1:200, and transferred to G-Rex100 culture bottles or breathable bags. Complete culture medium was replenished, samples were taken and counted every 1-3 days, and the medium was replenished or half-changed according to the cell status until the total number of cells was greater than 1×10⁹ or the second stage of in vitro expansion culture was about 5 days to about 14 days, after which the culture was terminated.

### 1.7 Harvest of tumor-infiltrating lymphocytes

The cells amplified in the second stage were taken, the culture medium supernatant was discarded after centrifugation, and they were washed three times with PBS, physiological saline, or compound electrolyte solution to obtain the TILs amplified in the second stage. Samples were taken and counted during the third wash. For counting results, the supernatant was discarded after the last centrifugation, cells were taken and sent for quality control testing; all remaining cells were added to cryopreservation solution, and the cell density was adjusted to 1-3×10⁸ cells/mL for cryopreservation.

### Example 6. TCR-T cell preparation

T cells from various sources such as peripheral blood and stem cell differentiation, preferably PBMC cells, were taken and cultured. The nucleic acid encoding the TCR was transferred into the above-mentioned T cells according to methods known in the art, such as viral transduction, LNP transduction, electroporation, etc. Thus TCR-T cells were obtained. Before or after TCR was transduced into T cells as described above, each test group was transduced with a retrovirus carrying a nucleic acid encoding mbIL-15 fusion protein (the amino acid sequence could be as shown in the present invention), and the transduced mbIL-15 fusion protein was obtained.

The sequence of the mbIL-15 fusion protein containing each group of linkers was, for example, SEQ ID NO: 57-62. Each group of TCR-T cells obtained after transduction were named PC163, PC228, PC241, PC242, PC243, and PC244, respectively. The fusion protein shown in SEQ ID NO: 57-58 contained GS linker as the control group; the fusion protein shown in SEQ ID NO: 59-60 contained linker2 shown in SEQ ID NO: 27 as the test group; and the fusion protein shown in SEQ ID NO: 61-62 included linker4 shown in SEQ ID NO: 29 as a test group.

T cells that were not transduced with mbIL-15 fusion protein were used as the untransduced control group (recorded as: Control).

### Example 7. MbIL-15 fusion protein promotes TCR-T cell proliferation

30 ng/mL anti-CD3 antibody (such as OKT3) was plated in a 96-well plate (those with antibody plating were recorded as CD3coating stimulation group, those without antibody plating were recorded as medium medium group), incubated at 37 degrees Celsius for 2 hours, and then the PBMC-TCR-T cells transduced with mbIL-15 fusion protein obtained in the Example were treated with 2 × 10⁵ cells/well on the 6th day after transduction. After 3 days, each group used a CTG kit to analyze cell expansion. According to the instructions of the CTG kit (CellTiter-Glo Luminescent Cell Viability Assay, Promega), the CTG reaction solution was prepared by mixing the CTG substrate (CellTiter-Glo Substrate) and the CTG buffer (CellTiter-Glo Buffer). The detection cell suspension was added into a 96-well microplate at 50 µL/well, and wells containing only culture medium were set as the background value of fluorescence. An equal volume of CTG reaction solution was added to each well, shaken on a horizontal shaker for 2 minutes, and let stand at room temperature for 10 minutes to stabilize the fluorescence signal, then the fluorescence value was read.

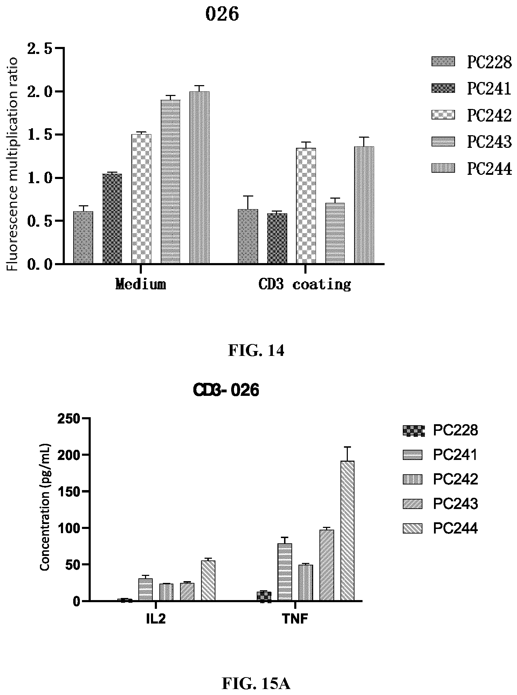

FIG. 14 shows the proliferation ability of T cells in each group transduced with the fusion protein of modified linker compared with the PC228 group transduced with the fusion protein of GS linker.

Compared with the PC228 group transduced with the GS linker control fusion protein, the T cell proliferation ability of each group transduced with the modified linker fusion protein was enhanced.

### Example 8. MbIL-15 fusion protein promotes PBMC-TCR-T cell factor secretion

30 ng/mL anti-CD3 was plated in a 96-well plate and incubated at 37 degrees Celsius for 2 hours. Then, the PBMC-TCR-T cells transduced with mbIL-15 fusion protein obtained in the above example were incubated at 1.5 × 10⁵ cells/well on the 6th day after transduction. The supernatant was taken after 24 hours, and the secretion of cytokines IL-2, TNF, and IFN-γ was analyzed using a Cytometric Bead Array (CBA) kit (BD).

For specific methods, the instructions of the cytokine detection kit were referred to. Specifically, the human Th1/Th2/Th17 cytokine standard freeze-dried powder was reconstituted with 2 mL of Assay Diluent diluent (the concentration of each cytokine in the standard stock solution was 5000 pg/mL) and serially diluted 1:2, 1:4, 1:8, 1:16, 1:32, 1:64, 1:128, 1:256, 1:512, 1:1024, labeled "Standard Tube". One tube containing only Assay Diluent was used as a reference. Each Capture Beads (BD) was added at 2 µL/Beads/well, then PE Detection Reagent was added at 10 µL/well and mixed to prepare a mixture, added at 22 µL/well into the V-bottom 96-well plate, and then 10 µL of each standard and the supernatant of the experimental group was added to each well, mixed, and incubated at room temperature in the dark for 3 hours. After the incubation, 200 µL Wash Buffer (BD) was added to each well and centrifuged at 500g for 3 minutes. After centrifugation, 100 µL Wash Buffer (BD) was added to each well to resuspend and analyzed by flow cytometry.

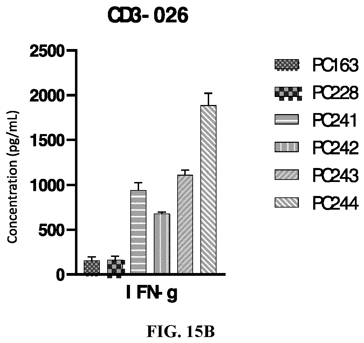

FIGs. 15A-15B show the ability of T cells transduced with the fusion protein of modified linker to secrete cytokines compared with the PC228 and/or PC163 group transduced with the GS linker fusion protein.

Compared with the PC228 and/or PC163 groups transduced with the GS linker control fusion protein, the ability of each group of T cells transduced with the fusion protein of modified linker to secrete cytokines was enhanced.

### Example 9. MbIL-15 fusion protein promotes the killing of tumor cells by PBMC-TCR-T

GFP-tagged A375 tumor cells (A375-GFP) were cultured at 2 × 10⁴/well in a 96-well plate. After the cells adhered, the PBMC-TCR-T cells obtained in the above examples that were transduced with the mbIL-15 fusion protein of the GS linker and the PBMC-TCR-T cells of each group that were transduced with the fusion protein of the modified linker were added to each well at 0.8 × 10⁴/well for co-culture. According to the instructions of the apoptosis detection reagent (Incucyte Caspase-3/7 Green Dye for Apoptosis, Sartorius), 0.2 µL/well Incucyte^{®} Caspase-3/7 Green Dye for Apoptosis was added, and culture medium was used to dilute Caspase 3/7 Green Dye at 25 µL/well. The activity of Caspase 3/7 was recorded using an Incucyte recorder (Sartorius) to analyze the killing ability of TILs on tumor cells. Recording was performed every 3 hours, and the total recording time was about 3 days.

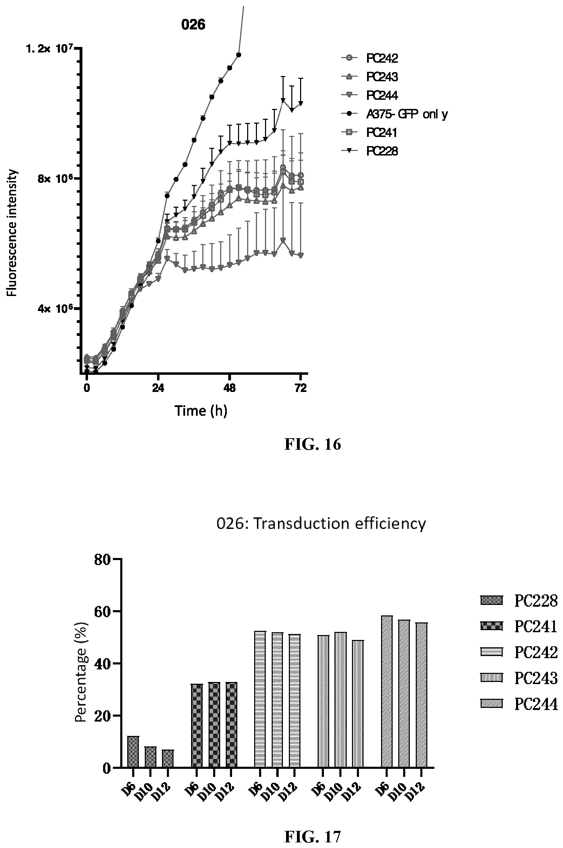

FIG. 16 shows the ability of each group of T cells transduced with mbIL-15 fusion protein to kill tumor cells.

T cells in each experimental group were able to kill tumor cells, and compared with the cells in the PC228 group transduced with the mbIL-15 fusion protein of the GS linker, the TCR-T cells transduced with the mbIL-15 fusion protein of the modified linker had enhanced tumor cell killing ability.

### Example 10. Effect of mbIL-15 fusion protein on changes in PBMC-TCR-T transduction efficiency

The PBMC-TCR-T cells transduced with the mbII,-15 fusion protein of the GS linker and the PBMC-TCR-T cells transduced with the mbII,-15 fusion protein of the modified linker obtained in the above examples were cultured continuously under medium conditions containing 30 IU/mL IL-2, and changes in transduction efficiency were detected by flow cytometry (Beckman Coulter). The day of recovery was recorded as D0, and the day of transduction was recorded as D3.

FIG. 17 shows the transduction efficiency of the mbIL-15 fusion protein containing the linker peptide of the present invention.
PC241, PC242, PC243, and PC244 of the mbIL-15 fusion protein containing the connecting peptide of the present invention could maintain stable transduction efficiency after transduction.

### Example 11. MbIL-15 fusion protein promotes TIL proliferation

The transduced TIL population obtained in step 1.5 of the above example was plated on a 96-well plate at a cell density of 2e5/well on the 9th day after transduction. T cells that were not transduced with the virus served as the untransduced control group. For the stimulation group, the stimulation condition was to add transACT (diameter about 100 to 500 nm, Miltenyi) at a volume ratio of 1: 1000. The unstimulated group was recorded as the medium medium group, and the stimulated group was marked as transact. After 3 days of stimulation, each group used a CTG kit to analyze cell expansion. The detection method of the CTG kit was as in Example 7. T cells that were not transduced with mbIL-15 fusion protein were used as the untransduced control group (recorded as: Control).

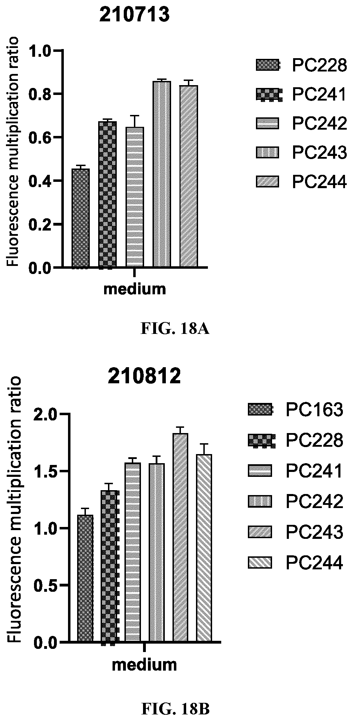

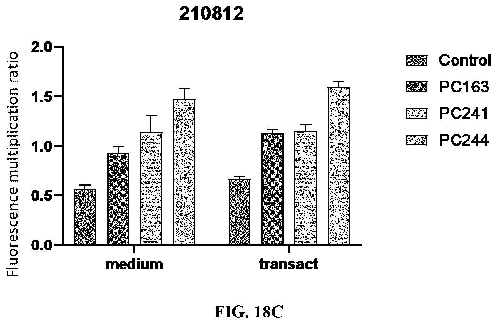

FIGs. 18A-18C show the proliferation ability of TIL transduced with mbIL-15 fusion protein. The proliferation ability of TIL transduced with mbIL-15 fusion protein modified with linker was stronger than that of the PC228 and/or PC163 groups transduced with GS linker.

### Example 12. MbIL-15 fusion protein promotes functional cytokine secretion from TILs

The transduced TIL population obtained in step 1.5 of Example 5 was treated with 2 × 10⁵ cells/well plated on a 96-well plate, and the unstimulated group was recorded as the medium medium group. After 24 hours, the supernatant was taken and the secretion of cytokines was analyzed using a CBA kit (BD) according to the instructions.

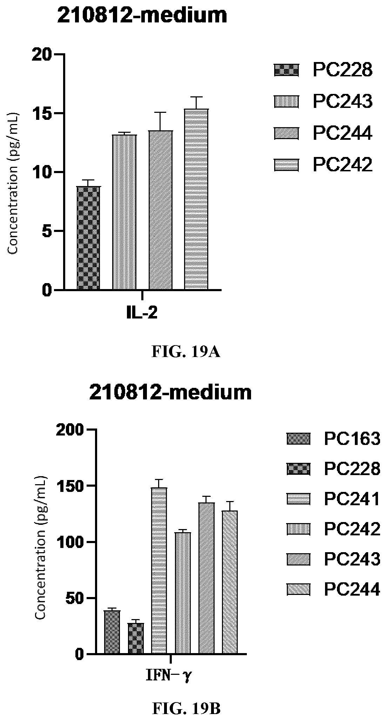

FIGs. 19A-19B show the ability of TIL transduced with mbIL-15 fusion protein to secrete the cytokines IL-2 and IFN-γ.

TILs (PC242, PC243, and PC244) had stronger ability to secrete cytokine IL-2 than the PC228 group of TIL cells transduced with GS linker. The ability of TIL transduced with mbIL-15 fusion protein of modified linker to secrete cytokine IFN-γ was stronger than that of the PC228 group transduced with GS linker TIL cells PC228 and/or PC163 group.

### Example 13. MbIL-15 fusion protein promotes TIL killing of tumor cells

A375 cells were spread at 2 × 10⁴/well in a 96-well plate. After the cells adhered, the TIL on the 7th day after transduction according to step 1.5 of Example 5 was inoculated with 2 × 10⁴/well added to each well for co-culture. According to the instructions of the apoptosis detection reagent (Incucyte Caspase-3/7 Green Dye for Apoptosis, Sartorius), 0.2 µL/well Incucyte^{®} Caspase-3/7 Green Dye for Apoptosis was added, and culture medium was used to dilute Caspase 3/7 Green Dye at 25 µL/well. The activity of Caspase 3/7 was recorded using an Incucyte recorder (Sartorius) to analyze the killing ability of TILs on tumor cells. Recording was performed every 3 hours, and the total recording time was about 3 days.

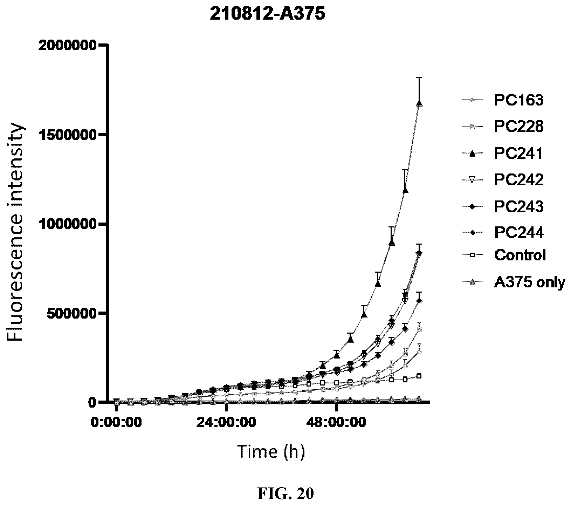

FIG. 20 shows the cell killing ability of TIL cells transduced with mbIL-15 fusion protein. TIL cells transduced with the linker-modified mbIL-15 fusion protein could have enhanced cell killing ability, and PC241, PC242, PC243, and PC244 had the strongest ability to kill tumor cells.

### Example 14. Effect of mbIL-15 fusion protein on changes in TIL transduction efficiency

After thawing, the TIL population activated according to step 1.4 of Example 5 was transduced according to step 1.5 of Example 5 on the second day, and feeder cells were added according to step 1.6 of Example 5 on the fourth day, under culture medium conditions containing 1000 IU/mL IL-2, 10 ng/mL IL-7, and 10 ng/mL IL-15. The cells were cultured continuously in the G-Rex24 culture system or 500 mL culture bottle (Innovil) culture system, and flow cytometry (Beckman Coulter) was used to detect changes in transduction efficiency. The day of recovery was recorded as D0, and the day of transduction was recorded as D2.

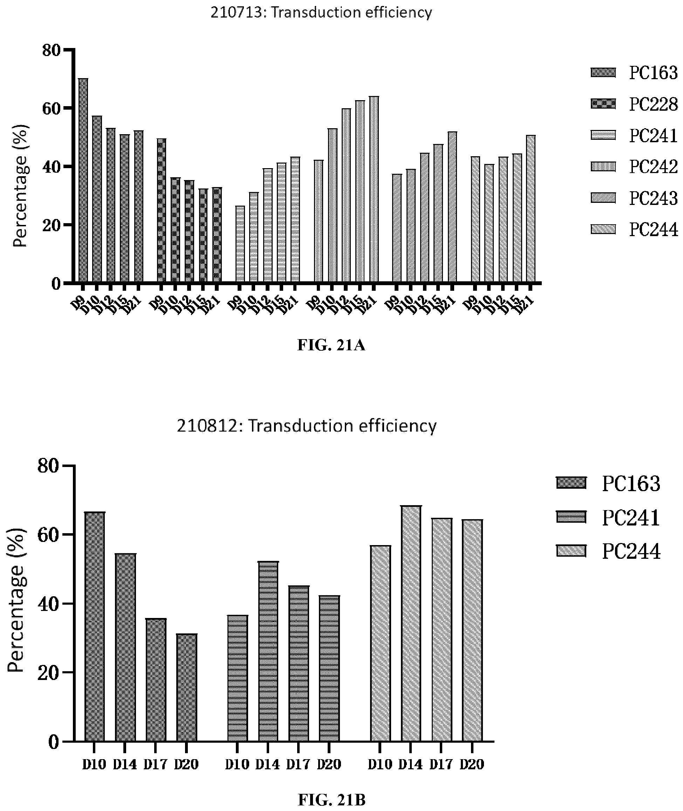

FIGs. 21A-21B show that in the G-Rex24 culture system (FIG. 21A) or under the 500 mL culture bottle (FIG. 21B), the mbIL-15 fusion protein containing the connecting peptide of the present invention could maintain stable transduction efficiency.

In the PC241, PC242, PC243, and PC244 groups that were transduced with the mbIL-15 fusion protein modified by the linker, the transduction efficiency did not decrease with the increase of culture time and even continued to increase or remained stable.

### Example 15. MbIL-15 fusion protein promotes STATS phosphorylation in TILs

After transduction according to step 1.5 of Example 5, the transduced TIL cells were resuspended in medium without cytokines on the 18th day after transduction. After one day of starvation, flow cytometry staining was performed. A kit was used to detect CD8/CD4 T cell ratio, IL-15 transduction efficiency, and STAT5 phosphorylation (pSTAT5) ratio in IL-15Rα negative and IL-15Rα positive T cells.

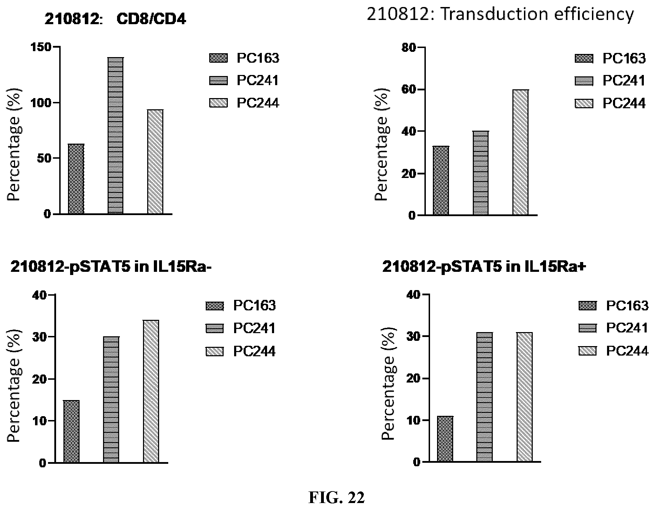

FIG. 22 shows that, compared with the PC163 group transduced with mbIL-15 fusion protein of GS linker, the ratio of CD8/CD4 T cells in TIL transduced with IL-15 fusion protein, the transduction efficiency, and the STAT5 phosphorylation ratio of IL-15Rα negative and IL-15Rα positive T cells. Compared with the PC163 group transduced with the GS linker control mbIL-15 fusion protein, TIL transduced with the modified linker had a higher ratio of CD8/CD4 T cells, high transduction efficiency, and had a higher STAT5 phosphorylation ratio in both IL-15Rα negative and IL-15Rα positive T cells.

### Example 16. Detection of fusion proteins containing connecting peptides with different proportions of strongly hydrophilic amino acids

The present invention provided a connecting peptide, which contained the number of strongly hydrophilic amino acids in a proportion of about 15% to 40% of the total number of all amino acids in the connecting peptide. For example, strongly hydrophilic amino acids included amino acids whose normalized hydrophobicity number was less than -0.4. For example, strongly hydrophilic amino acids included Glu(E), Asn(N), Gln(Q), Asp(D), Lys(K), and Arg(R). The connecting peptide was used to connect the interleukin-15 (IL-15) fragment and the interleukin-15 receptor alpha (IL-15Rα) fragment.

For the GS linker shown in SEQ ID NO: 25, the proportion of strongly hydrophilic amino acids was 3.85%; the group containing this linker was recorded as PC163.
For linker2 shown in SEQ ID NO: 27, the proportion of strongly hydrophilic amino acids was 30.77%; the group containing this linker was recorded as PC242.

For linker4 shown in SEQ ID NO: 29, the proportion of strongly hydrophilic amino acids was 34.62%; the group containing this linker was recorded as PC244.

For linker18 shown in SEQ ID NO: 43, the proportion of strongly hydrophilic amino acids was 19.23%; the group containing this linker was recorded as PC465.

For linker22 shown in SEQ ID NO: 47, the proportion of strongly hydrophilic amino acids was 25.00%; the group containing this linker was recorded as PC466.

For linker14 shown in SEQ ID NO: 39, the proportion of strongly hydrophilic amino acids was 36.00%; the group containing this linker was recorded as PC467.

### (1) TCR-T cell preparation

T cells from various sources such as peripheral blood and stem cell differentiation, preferably PBMC cells, were taken and cultured. The nucleic acid encoding the TCR was transferred into the above-mentioned T cells according to methods known in the art, such as viral transduction, LNP transduction, electroporation, etc. Thus TCR-T cells were obtained. Before, after, or at the same time as the TCR transduction of T cells as described above, each experimental group was transduced with a retrovirus carrying a nucleic acid encoding an mbIL-15 fusion protein containing a hydrophilic modified linker to obtain the transduced mbIL-15 fusion protein of TCR-T cells. TCR-T cells that were not transduced with mbIL-15 fusion protein were used as the untransduced control group (recorded as: TCR-T).

### (2) The proportion of strongly hydrophilic amino acids in the mbIL-15 fusion protein is increased, which promotes the long-term survival of TCR-T after IL-2 is removed

The PBMC-TCR-T cells obtained in the above examples and transduced with the mbIL-15 fusion protein with an increased proportion of strongly hydrophilic amino acids were cultured continuously in a medium without IL-2, and changes in viable cell numbers were detected by a cell counter (Chemometec).

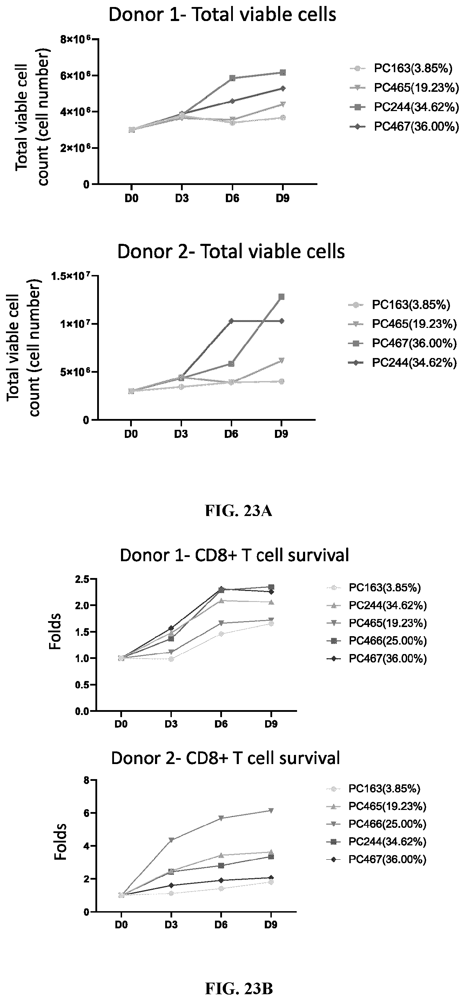

FIG. 23A shows the total number of viable cells transduced with each group of fusion proteins. The value in the parentheses of each group number represented the proportion of strongly hydrophilic amino acids in the linker used in that group.

PC465, PC467, and PC244 of the mbIL-15 fusion protein containing the connecting peptide of the present invention could maintain the long-term survival of cells after IL-2 was removed.

### (3) The proportion of strongly hydrophilic amino acids in the mbIL-15 fusion protein is increased, which promotes the removal of CD8 by TCR-T after IL-2 + T survives

The PBMC-TCR-T cells obtained in the above example and transduced with the mbIL-15 fusion protein with an increased proportion of strongly hydrophilic amino acids were cultured continuously in a medium without IL-2, and changes in the number of viable cells were detected every 3 days using a Chemometec instrument. CD8 + proportion of T cells was analyzed using flow cytometry (Beckman Coulter).

FIG. 23B shows that the CD8 + T cell survival ability of each group of cells transduced with the fusion protein. PC465, PC466, PC467, and PC244 of the mbIL-15 fusion protein containing the connecting peptide of the present invention could maintain CD8 + long-term survival of T cells after removing IL-2.

### (4) The proportion of strongly hydrophilic amino acids in the mbIL-15 fusion protein is increased, which promotes an increase in the proportion of transduced cells after TCR-T removes IL-2

The PBMC-TCR-T cells obtained in the above example and transduced with the mbIL-15 fusion protein with an increased proportion of strongly hydrophilic amino acids were cultured continuously in medium without IL-2, and the proportion of IL-15Rα+ cells was analyzed every 3 days using a flow cytometer (Beckman Coulter).

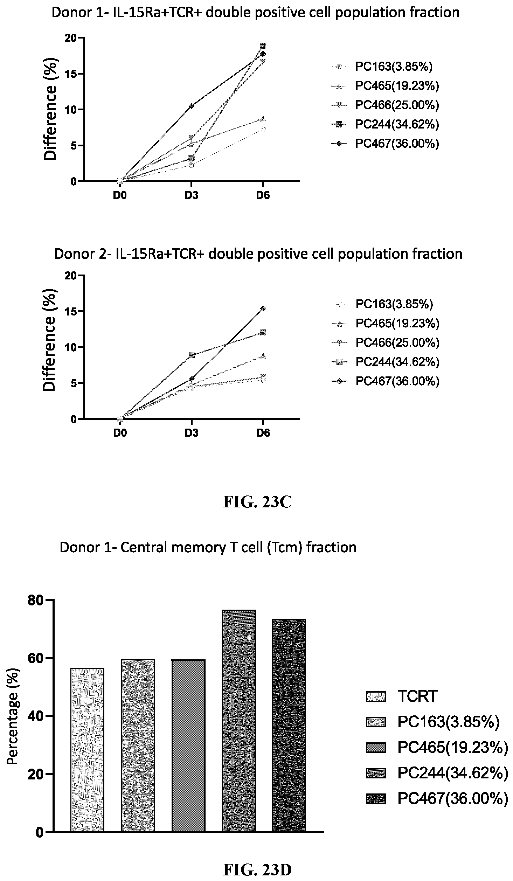

FIG. 23C shows the transduction efficiency of each group of cells transduced with the fusion protein. PC465, PC466, PC467, and PC244 of the mbIL-15 fusion protein containing the connecting peptide of the present invention could promote the proportion of transduced cells, namely IL15-Ra + TCR + double-positive cell populations, after removing IL-2.

### (5) Increasing the proportion of strongly hydrophilic amino acids in the mbIL-15 fusion protein to 34.62-36.00% promotes the increase in the Tcm phenotype of TCR-T

The PBMC-TCR-T cells obtained in the above examples and transduced with the mbIL-15 fusion protein with an increased proportion of strongly hydrophilic amino acids were analyzed by flow cytometry (Beckman Coulter) 7 days after transduction for central memory T cell proportion (Tcm, CD45RO + CD62L + ).

FIG. 23D shows the proportion of central memory T cell Tcm in each group of cells transduced with the fusion protein. PC467 and PC244 of the mbIL-15 fusion protein containing the connecting peptide of the present invention could promote the increase in the Tcm ratio.

### (6) The proportion of strongly hydrophilic amino acids in the mbIL-15 fusion protein is increased, which promotes the specific killing of A375-GFP target cells by TCR-T

The PBMC-TCR-T cells obtained in the above example and transduced with the mbIL-15 fusion protein with an increased proportion of strongly hydrophilic amino acids were transduced. Seven days after transduction, the tumor target cells A375-GFP were spread in a 96-well flat-bottom plate. The next day, T cells from each group were co-cultured with target cells at different effective-to-target ratios (T cells: target cells, E:T). 100 µL each of target cells and T cells were added, 4 duplicate wells were set in each group, and a control group containing only target cells was set. An Incucyte recorder (Sartorius) was used to record GFP autofluorescence to analyze the killing ability of T on target cells. Recording was performed once every 3 hours. After 3 days, tumor target cells A375-GFP were spread again in a 96-well flat bottom plate at 100 µL per well. Two hours later, when the tumor target cells were attached to the bottom of the well plate, each group of T cells was transferred to the well plate containing A375 at 100 µL per well, and an Incucyte recorder (Sartorius) was used to record GFP autofluorescence to analyze the killing ability of T cells on target cells. Recording was performed every 3 hours for a total of 4 days.

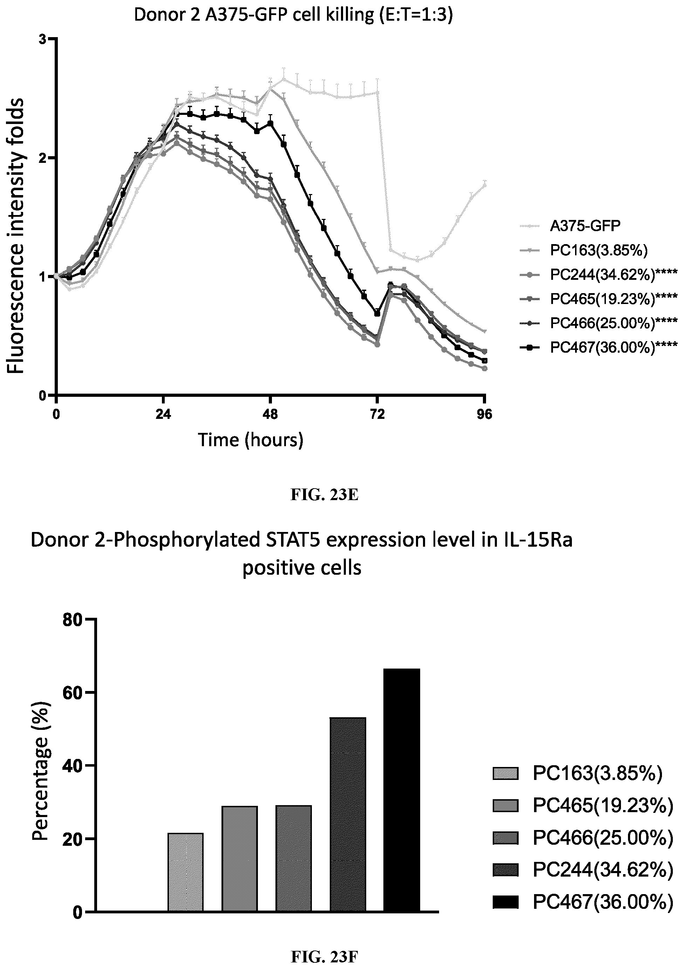

FIG. 23E shows the cell killing ability of each group of cells transduced with the fusion protein. PBMC-TCR-T cells transduced with mbIL-15 fusion protein with an increased proportion of strongly hydrophilic amino acids could have enhanced target cell killing ability. Relative to the significance of the group transduced with mbIL-15 fusion protein of GS linker, **** indicated p<0.0001.

### (7) The proportion of strongly hydrophilic amino acids in the mbIL-15 fusion protein is increased, which promotes the expression of TCR-T phosphorylated STAT5 (p-STAT5)

The PBMC-TCR-T cells obtained in the above example and transduced with the mbIL-15 fusion protein with an increased proportion of strongly hydrophilic amino acids were used. Eight days after transduction, 3E6/well cells were taken and adjusted with a medium without cytokines to a cell density of 1E6/mL, placed in a 6-well plate for culture. The cells were counted on day 1 and collected for p-STAT5 flow cytometry detection. First, live/dead dyes, anti-human CD3, CD4, CD8, and IL-15Rα were used to label the TCR-T cells transduced with mbIL-15 fusion protein, then fixed with Fixation buffer (Biolegend) and stored in ice-cold methanol. Flow Cytometry Staining Buffer (eBioscience) was used to prepare p-STAT5 antibodies to label p-STAT5 positive cells. The p-STAT5 positive proportion of TCR-T cells transduced with mbIL-15 fusion protein was analyzed using flow cytometry (Beckman Coulter).

FIG. 23F shows the p-STAT5 signal levels of cells in each group transduced with the fusion protein. The mbIL-15 fusion protein with an increased proportion of strongly hydrophilic amino acids was transduced, which promoted the increase in p-STAT5 expression in transduced cells.

### (8) The proportion of strongly hydrophilic amino acids in the mbIL-15 fusion protein is increased, which promotes the proliferation of TCR-T

The PBMC-TCR-T cells obtained in the above example and transduced with the mbIL-15 fusion protein with an increased proportion of strongly hydrophilic amino acids were treated with 2 × 10⁵ cells/well plated in a 96-well plate 8 days after transduction. Each group used a CTG kit (CellTiter-Glo Luminescent Cell Viability Assay, Promega) to analyze the cell expansion initially and after 3 days. According to the instructions of the CTG kit (CellTiter-Glo Luminescent Cell Viability Assay, Promega), the CTG reaction solution was prepared by mixing the CTG substrate (CellTiter-Glo Substrate) and the CTG buffer (CellTiter-Glo Buffer). The detection cell suspension was added into a 96-well microplate at 50 µL/well. An equal volume of CTG reaction solution was added to each well, shaken on a horizontal shaker for 2 minutes, and let stand at room temperature for 10 minutes to stabilize the fluorescence signal, then the fluorescence value was read.

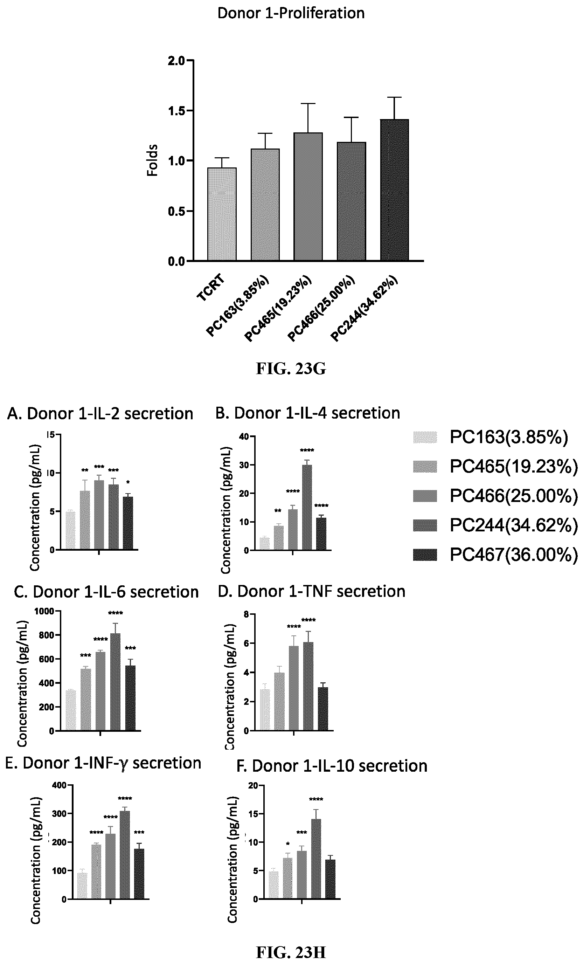

FIG. 23G shows the cell proliferation ability of each group of cells transduced with the fusion protein. PBMC-TCR-T cells with mbIL-15 fusion protein with an increased proportion of strongly hydrophilic amino acids had improved cell proliferation ability.

### (9) The proportion of strongly hydrophilic amino acids in the mbIL-15 fusion protein is increased, which promotes the secretion of TCR-T cytokines

The supernatant collected after the above co-incubation step was completed could be used for detection of cytokines such as IFN-γ. For the cytokine secretion detection method, the instructions of the cytokine detection kit (BD) were referred to. The human Th1/Th2/Th17 cytokine standard freeze-dried powder (BD) was reconstituted with 2 mL Assay Diluent diluent (BD) (the concentration of each cytokine in the standard solution was 5000 pg/mL) and serially diluted 1:2, 1:4, 1:8, 1:16, 1:32, 1:64, 1:128, 1:256, 1:512, 1:1024, labeled "standard tube". One tube containing only Assay Diluent (test diluent) was used as a reference. Each Capture Beads (BD) was added at 2 µL/Beads/well, then PE Detection Reagent (BD) was added at 10 µL/well and mixed to prepare a mixture, added at 22 µL/well into the V-bottom 96-well plate. Each standard and the supernatant of the experimental group was added at 10 µL/well, mixed, and incubated at room temperature in the dark for 3 hours. At the end of the incubation, 200 µL Wash Buffer (BD) was added to each well and centrifuged at 500g for 3 minutes. After centrifugation, 100 µL Wash Buffer (BD) was added to each well to resuspend and analyzed by flow cytometry.

FIG. 23H shows the cytokine release ability of each group of cells transduced with the fusion protein. PBMC-TCR-T cells transduced with mbIL-15 fusion protein with an increased proportion of strongly hydrophilic amino acids promoted the improvement of TCR-T cytokine secretion ability.

### Example 17. Fusion proteins containing strongly hydrophilic amino acid linking peptides are used in various immune cells

The present invention provides a connecting peptide, which contained the number of strongly hydrophilic amino acids in a proportion of about 15% to 40% of the total number of all amino acids in the connecting peptide. For example, strongly hydrophilic amino acids included amino acids whose normalized hydrophobicity number was less than -0.4. For example, strongly hydrophilic amino acids included Glu(E), Asn(N), Gln(Q), Asp(D), Lys(K), and Arg(R). The connecting peptide was used to connect the interleukin-15 (IL-15) fragment and the interleukin-15 receptor alpha (IL-15Rα) fragment.

For the GS linker shown in SEQ ID NO: 25, the proportion of strongly hydrophilic amino acids was 3.85%; the group containing this linker was recorded as PC163.

For linker2 shown in SEQ ID NO: 27, the proportion of strongly hydrophilic amino acids was 30.77%; the group containing this linker was recorded as PC242.

For linker4 shown in SEQ ID NO: 29, the proportion of strongly hydrophilic amino acids was 34.62%; the group containing this linker was recorded as PC244.

For linker18 shown in SEQ ID NO: 43, the proportion of strongly hydrophilic amino acids was 19.23%; the group containing this linker was recorded as PC465.

For linker22 shown in SEQ ID NO: 47, the proportion of strongly hydrophilic amino acids was 25.00%; the group containing this linker was recorded as PC466.

For linker14 shown in SEQ ID NO: 39, the proportion of strongly hydrophilic amino acids was 36.00%; the group containing this linker was recorded as PC467.

Referring to the method of Example 16, NK cells were transduced with a retrovirus carrying a nucleic acid encoding an mbIL-15 fusion protein containing a hydrophilic modified linker to obtain NK cells transduced with the mbIL-15 fusion protein. NK cells that were not transduced with mbIL-15 fusion protein were used as the untransduced control group (recorded as: NK). The group with only K562 target cells added was recorded as tumor control. The method of Example 16 was referred to detect NK cells in each group.

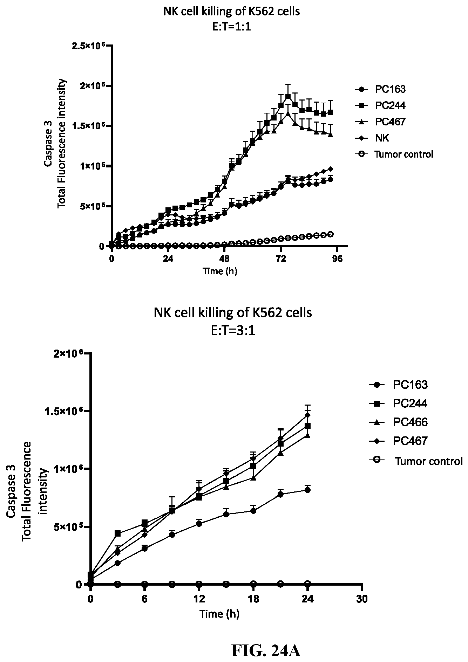

FIG. 24A shows the cell killing ability of each group of cells transduced with the fusion protein. Compared with the control group PC163 group transduced with GS linker or the untransduced NK group, NK cells transduced with mbIL-15 fusion protein with an increased proportion of strongly hydrophilic amino acids could have enhanced cell killing ability.

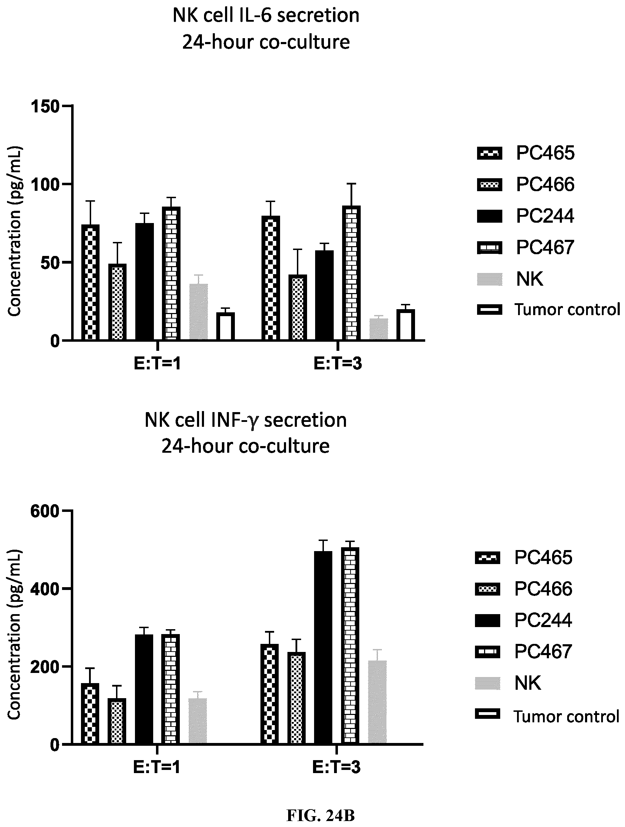

FIG. 24B shows the cytokine release ability of each group of cells transduced with the fusion protein. Compared with the non-transduced NK group, NK cells transduced with mbIL-15 fusion protein with an increased proportion of strongly hydrophilic amino acids could have enhanced cytokine release capacity.

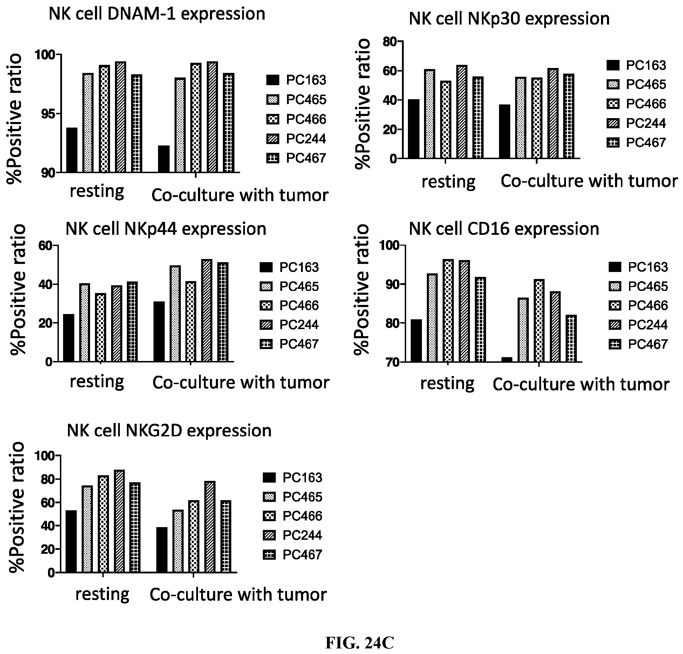

FIG. 24C shows the surface marker expression of each group of cells transduced with the fusion protein. Compared with the PC163 group transduced as a control group containing GS linker, NK cells transduced with mbIL-15 fusion protein with an increased proportion of strongly hydrophilic amino acids could have a more favorable cell subpopulation phenotype, such as higher DNAM-1 expression, higher NKp30 expression, higher NKp44 expression, higher CD16 expression, and higher NKG2D expression.

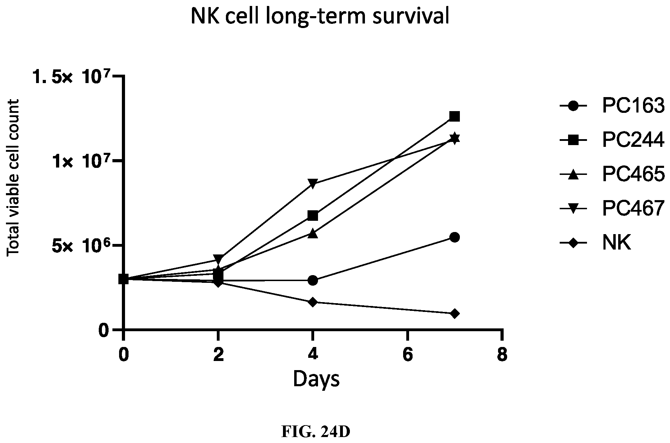

FIG. 24D shows the cell survival ability of each group of cells transduced with the fusion protein. Compared with the control group PC163 group or the non-transduced NK group transduced with the GS linker, NK cells transduced with the mbIL-15 fusion protein with an increased proportion of strongly hydrophilic amino acids could have enhanced long-term cell survival.

The foregoing detailed description is provided by way of explanation and example, and is not intended to limit the scope of the appended claims. Various modifications to the presently illustrated embodiments of the invention will be apparent to those skilled in the art and remain within the scope of the appended claims and their equivalents.

## Claims

1. A fusion polypeptide comprising a mutated interleukin-15 (IL-15) receptor or a functional fragment thereof, wherein the percentage of membrane-bound expression product of the fusion polypeptide comprising the mutated interleukin-15 receptor is increased in a cell after intracellular expression, comparing to a protein comprising a native interleukin-15 receptor.

2. The fusion polypeptide of claim 1, wherein the mutated interleukin-15 receptor comprises a mutated transmembrane domain of an IL-15 receptor α subunit (IL-15Rα), and the mutated transmembrane domain comprises at least one amino acid mutation compared to the transmembrane domain of a native IL-15 receptor α subunit.

3. The fusion polypeptide of claim 2, wherein the mutated transmembrane domain comprises at least one amino acid mutation at positions 3 to 6 compared to the transmembrane domain VAISTSTVLLCGLSAVSLLACYL (SEQ ID NO: 17) of the native IL-15 receptor α subunit.

4. The fusion polypeptide of any one of claims 1-3, wherein the mutation comprises a deletion mutation.

5. The fusion polypeptide of any one of claims 1-4, wherein the mutated IL-15 receptor comprises a sequence selected from any one of SEQ ID NOs: 1-4.

6. The fusion polypeptide of any one of claims 1-5, further comprising an IL-15 structural domain.

7. The fusion polypeptide of claim 6, wherein the IL-15 structural domain comprises the amino acid sequence shown in SEQ ID NO: 11.

8. The fusion polypeptide of any one of claims 1-7, further comprising the extracellular domain of an IL-15 receptor.

9. The fusion polypeptide of claim 8, wherein the extracellular domain of the IL-15 receptor comprises the extracellular domain of IL-15Rα, and the extracellular domain of IL-15Rα comprises the amino acid sequence shown in SEQ ID NO: 13.

10. The fusion polypeptide of any one of claims 1-9, further comprising the intracellular domain of the IL-15 receptor.

11. The fusion polypeptide of claim 10, wherein the extracellular domain of the IL-15 receptor comprises the intracellular domain of IL-15Rα, and the intracellular domain of IL-15Rα comprises the amino acid sequence as shown in SEQ ID NO: 15.

12. The fusion polypeptide of any one of claims 6-11, wherein the IL-15 structural domain is directly or indirectly linked to the IL-15 receptor or functional fragment thereof.

13. The fusion polypeptide of claim 12, wherein said indirect linkage comprises linking via a linker peptide.

14. The fusion polypeptide of claim 13, wherein the linker peptide comprises an amino acid sequence selected from SEQ ID NO: 7, SEQ ID NO: 8, and SEQ ID NO: 12.

15. The fusion polypeptide of any one of claims 1-14, further comprising a signal peptide.

16. The fusion polypeptide of claim 15, wherein the signal peptide comprises an amino acid sequence selected from SEQ ID NO: 5, SEQ ID NO: 6, and SEQ ID NO: 10.

17. The fusion polypeptide of any one of claims 1-16, comprising the amino acid sequence shown in SEQ ID NO: 9.

18. A nucleic acid encoding the fusion polypeptide of any one of claims 1-17.

19. A vector comprising the nucleic acid of claim 18.

20. A cell comprising the fusion polypeptide of any one of claims 1-17, the nucleic acid of claim 18, and/or the vector of claim 19.

21. The cell of claim 20, comprising an immune cell.

22. The cell of any one of claims 20-21, comprising NK cells, NKT cells and/or T cells.

23. The cell of any one of claims 20-22, comprising cells derived from induced pluripotent stem cells (iPSC), PBMC cells and/or tumor-infiltrating lymphocytes.

24. A method for preparing the fusion polypeptide of any one of claims 1-17, said method comprising culturing the cell of any one of claims 20-23 under conditions such that the fusion polypeptide is expressed.

25. A composition comprising the fusion polypeptide of any one of claims 1-17, the nucleic acid of claim 18, the vector of claim 19, and/or the cell of any one of claims 20-23, and optionally a pharmaceutically acceptable carrier.

26. A kit comprising the fusion polypeptide of any one of claims 1-17, the nucleic acid of claim 18, the vector of claim 19, the cell of any one of claims 20-23 and/or the composition of claim 25.

27. A method for culturing cells, comprising providing the fusion polypeptide of any one of claims 1-17, the nucleic acid of claim 18, the vector of claim 19, and the cell of any one of claims 20-23, the composition of claim 25, and/or the kit of claim 26.

28. Use of a fusion polypeptide of any one of claims 1-17, a nucleic acid of claim 18, a vector of claim 19, a cell of any one of claims 20-23, the composition of claim 25 and/or the kit of claim 26 in the preparation of a medicament for preventing, alleviating and/or treating a disease (preferably a tumor).

29. A medicament for use to prevent and/or treat a disease and/or a symptom (preferably a tumor), comprising the fusion polypeptide of any one of claims 1-17, the nucleic acid of claim 18, the vector of claim 19, the cell of any one of claims 20-23, the composition of claim 25, and/or the kit of claim 26.

30. A method for preventing and/or treating a disease and/or a symptom (preferably a tumor), comprising administering to a subject in need thereof the fusion polypeptide of any one of claims 1-17, the nucleic acid of claim 18, the vector of claim 19, the cell of any one of claims 20-23, the composition of claim 25, and/or the kit of claim 26.

31. The fusion polypeptide of any one of claims 1-17, the nucleic acid of claim 18, the vector of claim 19, the cell of any one of claims 20-23, the composition of claim 25, and/or the kit of claim 26, useful in preventing and/or treating a disease and/or a symptom (preferably a tumor).
